(19) Europäisches Patentamt
European Patent Office
Office européen des brevets

(11) Veröffentlichungsnummer: **0 352 581**
**A2**

# (12) EUROPÄISCHE PATENTANMELDUNG

(21) Anmeldenummer: **89113031.2**

(22) Anmeldetag: **15.07.89**

(51) Int. Cl.⁴: **C07D 277/56 , C07D 263/34 , A61K 31/425 , C07D 261/18 , C07D 231/14 , A61K 31/42 , C07D 417/04 , C07D 413/04 , C07D 263/48**

(30) Priorität: **28.07.88 CH 2871/88**

(43) Veröffentlichungstag der Anmeldung:
**31.01.90 Patentblatt 90/05**

(84) Benannte Vertragsstaaten:
**AT BE CH DE FR GB IT LI LU NL SE**

(71) Anmelder: **F. HOFFMANN-LA ROCHE AG**
**Postfach 3255**
**CH-4002 Basel(CH)**

(72) Erfinder: **Gassner, Walter**
**Zehntenfreistrasse 31**
**CH-4103 Bottmingen(CH)**
Erfinder: **Imhof, René, Dr.**
**Bleumatthöhe 3**
**CH-5264 Gipf-Oberfrick(CH)**
Erfinder: **Kyburz, Emilio, Dr.**
**Unterer Rebbergweg 127**
**CH-4153 Reinach(CH)**

(74) Vertreter: **Kellenberger, Marcus, Dr. et al**
**Grenzacherstrasse 124 Postfach 3255**
**CH-4002 Basel(CH)**

## (54) Aethylendiaminmonoamid-Derivate.

(57) Die Aethylendiaminmonoamide der Formel

$$R\text{-}CO\text{-}NH\text{-}CH_2\text{-}CH_2\text{-}NH_2 \quad I$$

worin R einen aromatischen, 5-gliedrigen heterocyclischen Rest wie er in Anspruch 1 defininiert ist, bedeutet, sowie deren pharmazeutisch verwendbare Säureadditionssalze besitzen bei geringer Toxizität interessante Monoaminooxidase hemmende Eigenschaften und können demnach zur Behandlung von depressiven Zuständen und kognitiven Erkrankungen verwendet werden. Sie können nach bekannten Methoden hergestellt werden.

EP 0 352 581 A2

## Aethylendiaminmonoamid-Derivate

Die vorliegende Erfindung betrifft Aethylendiaminmonoamid-Derivate. Im speziellen betrifft sie Aethylendiaminmonoamide der allgemeinen Formel

$R-CO-NH-CH_2-CH_2-NH_2$   I

worin R eine der Gruppen

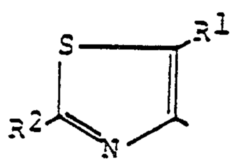

(a)

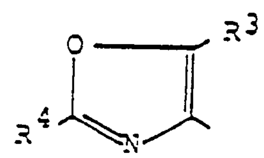

(b)

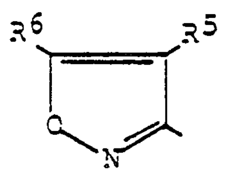

(c)

und

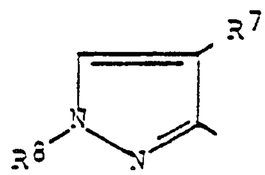

(d)

bedeutet, worin $R^1$ gegebenenfalls durch Halogen, nieder- Alkyl, nieder-Alkoxy, Trifluormethyl, Cyan oder Aryl-nieder-alkoxy monosubstituiertes Phenyl, Dihalophenyl, Furyl oder gegebenenfalls durch Halogen monosubstituiertes Thienyl, $R^2$ Wasserstoff, Halogen oder Amino, $R^3$, $R^5$ und $R^7$ je gegebenenfalls durch Halogen mono-oder di-substituiertes Phenyl, Thienyl oder gegebenenfalls durch Halogen monosubstituiertes Furyl, $R^4$ und $R^6$ je Wasserstoff oder Amino und $R^8$ Wasserstoff oder nieder-Alkyl bedeuten,
sowie pharmazeutisch verwendbare Säureadditionssalze davon.

Im Rahmen der vorliegenden Erfindung wurde überraschenderweise gefunden, dass die Verbindungen der Formel I bei geringer Toxizität interessante und therapeutisch verwertbare pharmakodynamische Eigenschaften aufweisen. In Tierversuchen konnte nämlich gezeigt werden, dass die Verbindungen der obigen Formel I sowie ihre pharmazeutisch verwendbaren Säureadditionssalze Monoaminooxidase (MAO) hemmende Eigenschaften besitzen.

Gegenstand der vorliegenden Erfindung sind die Verbindungen der allgemeinen Formel I sowie ihre pharmazeutisch verwendbaren Säureadditionssalze per se und als pharmazeutische Wirkstoffe, Arzneimittel, enthaltend eine Verbindung der allgemeinen Formel I oder ein pharmazeutisch verwendbares Säureadditionssalz davon, die Herstellung solcher Arzneimittel und die Verwendung von Verbindungen der allgemeinen Formel I sowie ihrer pharmazeutisch verwendbaren Säureadditionssalze bei der Bekämpfung bzw. Verhütung von Krankheiten bzw. bei der Verbesserung der Gesundheit, insbesondere bei der Bekämpfung bzw. Verhütung von depressiven Zuständen und kognitiven Erkrankungen, insbesondere von solchen, die altersbedingt sind. Gegenstand vorliegender Erfindung ist schliesslich ein Verfahren zur Herstellung der Verbindungen der obigen Formel I und ihrer pharmazeutisch verwendbaren Säureadditionssalze sowie die in diesem Verfahren eingesetzten Zwischenprodukte.

Der in dieser Beschreibung verwendete Ausdruck "nieder-Alkyl" bezieht sich auf geradkettige und verzweigte Kohlenwasserstoffreste mit 1-3 Kohlenstoffatomen, d.h. Methyl, Aethyl, n-Propyl und Isopropyl. Der Ausdruck "nieder-Alkoxy" bezieht sich auf niedere Alkyläthergruppen, in denen der Ausdruck "nieder-Alkyl" die obige Bedeutung besitzt. Der Ausdruck "Aryl-nieder-Alkoxy" bezieht sich auf niedere Alkyläthergruppen, in denen ein Wasserstoffatom durch einen gegebenenfalls durch Halogen, nieder-Alkyl, nieder-Alkoxy, Nitro, Cyan oder Hydroxy substituierten Phenylrest ersetzt ist. Der Ausdruck "Halogen" umfasst die vier Halogene Fluor, Chlor, Brom und Jod. Der Ausdruck "Abgangsgruppe" bedeutet im Rahmen der vorliegenden Erfindung bekannte Gruppen, wie Halogen, vorzugsweise Chlor oder Brom, Arylsulfonyloxy, wie etwa Tosyloxy, Alkylsulfonyloxy, wie etwa Mesyloxy, und dergleichen.

Der Ausdruck "pharmazeutisch verwendbare Säureadditionssalze" umfasst Salze mit anorganischen

und organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Bevorzugte Verbindungen der Formel I sind solche, worin R die Gruppe (a) oder (b) bedeutet.

Bevorzugt sind dabei solche Verbindungen, worin $R^1$ gegebenenfalls durch Halogen, nieder-Alkyl, nieder-Alkoxy, Trifluormethyl oder Cyan monosubstituiertes Phenyl, Dihalophenyl, Furyl oder gegebenenfalls durch Halogen monosubstituiertes Thienyl und $R^2$ Wasserstoff oder Amino bedeuten, bzw. solche Verbindungen, worin $R^3$ durch Halogen mono- oder di-substituiertes Phenyl, Thienyl oder gegebenenfalls durch Halogen monosubstituiertes Furyl und $R^4$ Wasserstoff bedeuten.

Besonders bevorzugt sind diejenigen Verbindungen, worin $R^1$ durch Halogen monosubstituiertes Phenyl oder Dihalophenyl und $R^2$ Wasserstoff bedeuten bzw. diejenigen Verbindungen, worin $R^3$ durch Halogen mono- oder di-substituiertes Phenyl und $R^4$ Wasserstoff bedeuten.

Aus dem obigen folgt, dass von den Verbindungen der Formel I diejenigen besonders bevorzugt sind, worin R die Gruppe (a) oder (b) bedeutet, $R^1$ durch Halogen monosubstituiertes Phenyl oder Dihalophenyl und $R^2$ Wasserstoff bedeuten bzw. $R^3$ durch Halogen mono- oder di-substituiertes Phenyl und $R^4$ Wasserstoff bedeuten.

Ganz besonders bevorzugte Verbindungen der Formel I sind:

N-(2-Aminoäthyl)-5-phenyl-4-thiazolcarboxamid
N-(2-Aminoäthyl)-5-(2-fluorphenyl)-4-thiazolcarboxamid
N- 2-Aminoäthyl)-5-(3-fluorphenyl)-4-thiazolcarboxamid
N-(2-Aminoäthyl)-5-(4-fluorphenyl)-4-thiazolcarboxamid
N-(2-Aminoäthyl)-5-(4-chlorphenyl)-4-oxazolcarboxamid
N-(2-Aminoäthyl)-5-(2-furyl)-4-oxazolcarboxamid
N-(2-Aminoäthyl)-5-(3,5-dichlorphenyl)-4-thiazolcarboxamid
N-(2-Aminoäthyl)-5-(2,4-difluorphenyl)-4-thiazolcarboxamid
N-(2-Aminoäthyl)-5-(3,5-difluorphenyl)-4-thiazolcarboxamid
N-(2-Aminoäthyl)-5-(4-fluorphenyl)-4-oxazolcarboxamid
N-(2-Aminoäthyl)-5-(4-bromphenyl)-4-oxazolcarboxamid
N-(2-Aminoäthyl)-4-(3-fluorphenyl)-3-isoxazolcarboxamid und
N-(2-Aminoäthyl)-4-(3-fluorphenyl)-1-methyl-3-pyrazolcarboxamid.

Die Verbindungen der Formel I und deren pharmazeutisch verwendbare Säureadditionssalze lassen sich erfindungsgemäss herstellen, indem man

a) eine Verbindung der allgemeinen Formel

R-CO-OH II

worin R obige Bedeutung hat,
in Form der freien Säure oder in Form eines reaktionsfähigen funktionellen Derivates davon mit Aethylendiamin umsetzt, oder

b) eine Verbindung der allgemeinen Formel

$R-CO-NH-CH_2-CH_2-R^9$ III

worin R obige Bedeutung hat und $R^9$ eine Abgangsgruppe bedeutet, mit Ammoniak umsetzt, oder

c) in einer Verbindung der allgemeinen Formel

$R-CO-NH-CH_2-CH_2-R^{10}$ IV

worin R obige Bedeutung hat und $R^{10}$ einen in eine Aminogruppe überführbaren Rest bedeutet, den Rest $R^{10}$ in die Aminogruppe überführt, und erwünschtenfalls, eine erhaltene Verbindung in ein pharmazeutisch verwendbares Säureadditionssalz überführt.

Als reaktionsfähige funktionelle Derivate der Säuren der Formel II kommen beispielsweise Halogenide, z.B. Chloride, symmetrische oder gemischte Anhydride, Ester, z.B. Methylester, p-Nitrophenylester oder N-Hydroxysuccinimidester, Azide und Amide, z.B. Imidazolide oder Succinimide, in Betracht.

Die Umsetzung einer Säure der Formel II oder eines reaktionsfähigen funktionellen Derivates davon mit Aethylendiamin nach Variante a) des obigen Verfahrens, kann man nach üblichen Methoden durchführen. So kann man z.B. eine freie Säure der Formel II mit Aethylendiamin in Gegenwart eines Kondensationsmittels in einem inerten Lösungsmittel umsetzen. Wird ein Carbodiimid, wie 1,1′-Carbonyldiimidazol oder Dicyclohexycarbodiimid, als Kondensationsmittel verwendet, so wird die Umsetzung zweckmässig in einem Alkancarbonsäureester, wie Aethylacetat, einem Aether, wie Tetrahydrofuran oder Dioxan, einem chlorierten Kohlenwasserstoff, wie Methylenchlorid oder Chloroform, einem aromatischen Kohlenwasserstoff, wie Benzol, Toluol oder Xylol, Acetonitril oder Dimethylformamid, bei einer Temperatur zwischen etwa 0°C und

etwa 100 °C, vorzugsweise bei etwa 60 °C, durchgeführt. Wird Phosphortrichlorid als Kondensationsmittel verwendet, so wird die Umsetzung zweckmässig in einem Lösungsmittel, wie Pyridin, bei einer Temperatur zwischen etwa 0 °C und der Rückflusstemperatur des Reaktionsgemisches durchgeführt. In einer anderen Ausführungsform der Variante a) wird Aethylendiamin mit einem der weiter oben genannten reaktionsfähigen funktionellen Derivate einer Säure der Formel II umgesetzt. So kann man z.B. eine Halogenid, z.B. das Chlorid, einer Säure der Formel II bei etwa 0 °C mit Aethylendiamin in Gegenwart eines Lösungsmittels, wie z.B. Methylenchlorid oder Aether, umsetzen.

Die Verbindungen der Formel III sind beispielsweise N-(2-Haloäthyl)carboxamide, wie N-(2-Chloräthyl)-carboxamide, N-(2-Methylsulfonyläthyl)carboxamide oder N-(2-p-Toluolsulfonyläthyl)carboxamide und dergleichen.

Gemäss Variante b) kann man in an sich bekannter Weise eine Verbindung der Formel III mit Ammoniak bei einer Temperatur zwischen etwa -40 °C und 50 °C, erwünschtenfalls in Gegenwart eines Lösungsmittels, wie Dimethylformamid, Dimethylacetamid, Dimethylsulfoxyd und dergleichen, umsetzen. Zweckmässigerweise arbeitet man in Gegenwart eines Lösungs mittels bei etwa Raumtemperatur.

Die Ueberführung des Restes $R^{10}$ in Amino gemäss Variante c) erfolgt ebenfalls in an sich bekannter Weise in Abhängigkeit von der Art des Restes $R^{10}$. Ist dieser ein Amid, so erfolgt die Ueberführung zweckmässig durch saure oder basische Hydrolyse. Für die saure Hydrolyse verwendet man vorteilhafterweise eine Lösung einer Mineralsäure, wie Salzsäure, wässriger Bromwasserstoff, Schwefelsäure, Phosphorsäure und dergleichen, in einem inerten Lösungsmittel, wie einem Alkohol, z.B. Methanol oder Aethanol, einem Aether, z.B. Tetrahydrofuran oder Dioxan, und dergleichen. Für die basische Hydrolyse können wässrige Lösungen von Alkalimetallhydroxyden, wie Kali- oder Natronlauge, verwendet werden. Inerte organische Lösungsmittel, wie sie oben für die saure Hydrolyse genannt sind, können als Lösungsvermittler zugegeben werden. Die Reaktionstemperatur kann für die saure und basische Hydrolyse in einem Bereich von etwa Raum- bis Rückflusstemperatur variiert werden, wobei man vorzugsweise bei der Siedetemperatur des Reaktionsgemisches oder wenig darunter arbeitet. Ist $R^{10}$ Phthalimido, so kann neben der sauren und basischen Hydrolyse auch eine Aminolyse mit einer wässrigen Lösung eines niederen Alkylamins, wie Methylamin oder Aethylamin, durchgeführt werden. Als organisches Lösungsmittel kann man ein niederes Alkanol, wie Aethanol, verwenden. Die Reaktion wird vorzugsweise bei Raumtemperatur durchgeführt. Eine dritte Methode zur Ueberführung von Phthalimido in Amino besteht darin, dass man Verbindungen der Formel IV mit Hydrazin in einem inerten Lösungsmittel, wie Aethanol, einer Mischung aus Aethanol und Chloroform, Tetrahydrofuran oder wässrigem Aethanol umsetzt. Die Reaktionstemperatur kann in einem Bereich von etwa Raumtemperatur bis etwa 100 °C variiert werden, wobei man vorzugsweise bei der Siedetemperatur des gewählten Lösungsmittels arbeitet. Das entstandene Produkt kann mit verdünnter Mineralsäure ausgeschüttelt werden und anschliessend durch Basischstellen der sauren Lösung erhalten werden. Der t-Butoxycarbonylaminorest wird zweckmässig mit Trifluor essigsäure oder Ameisensäure in Gegenwart oder Abwesenheit eines inerten Lösungsmittels, wie Methylenchlorid, bei etwa Raumtemperatur in die Aminogruppe überführt, während die Ueberführung der Trichloräthoxycarbonylaminogruppe mit Zink oder Cadmium unter sauren Bedingungen erfolgt. Die sauren Bedingungen werden zweckmässig dadurch erreicht, dass man die Reaktion in Essigsäure in Gegenwart oder Abwesenheit eines zusätzlichen inerten Lösungsmittels, wie eines Alkohols, z.B. Methanol, durchführt. Der Benzyloxycarbonylaminorest kann in bekannter Weise durch saure Hydrolyse wie weiter oben beschrieben oder hydrogenolytisch in die Aminogruppe überführt werden. Eine Azidogruppe kann nach bekannten Methoden, beispielsweise mit elementarem Wasserstoff in Gegenwart eines Katalysators, wie Palladium/Kohle, Raney-Nickel, Platinoxid und dergleichen, zur Aminogruppe reduziert werden. Eine Hexamethylentetrammoniumgruppe kann nach ebenfalls bekannten Methoden durch saure Hydrolyse in die Aminogruppe überführt werden.

Die in Variante a) als Ausgangsstoffe verwendeten Verbindungen der Formel II bzw. deren reaktionskähige funktionelle Derivate sind neu, gehören jedoch bekannten Stoffklassen an und können in Analogie zur Herstellung von bekannten Verbindungen erhalten werden. Sie sind ebenfalls Gegenstand vorliegender Erfindung.

Verfahren zur Herstellung je eines Verbindungstyps der Formel II, worin R die Gruppe (a), (b), (c) oder (d) bedeutet, sind in den nachfolgenden Schemata I und II, worin $R^8$ Wasserstoff oder nieder-Alkyl und $R^{11}$ Wasserstoff, Halogen, nieder-Alkyl, nieder-Alkoxy, Trifluormethyl, Cyan oder Aryl-nieder-alkoxy, $R^{12}$ nieder-Alkyl $R^{13}$ Wasserstoff oder Halogen und X Halogen bedeuten, skizziert. Bezüglich der genauen Reaktionsbedingungen wird auf den Beispielteil verwiesen.

Schema I

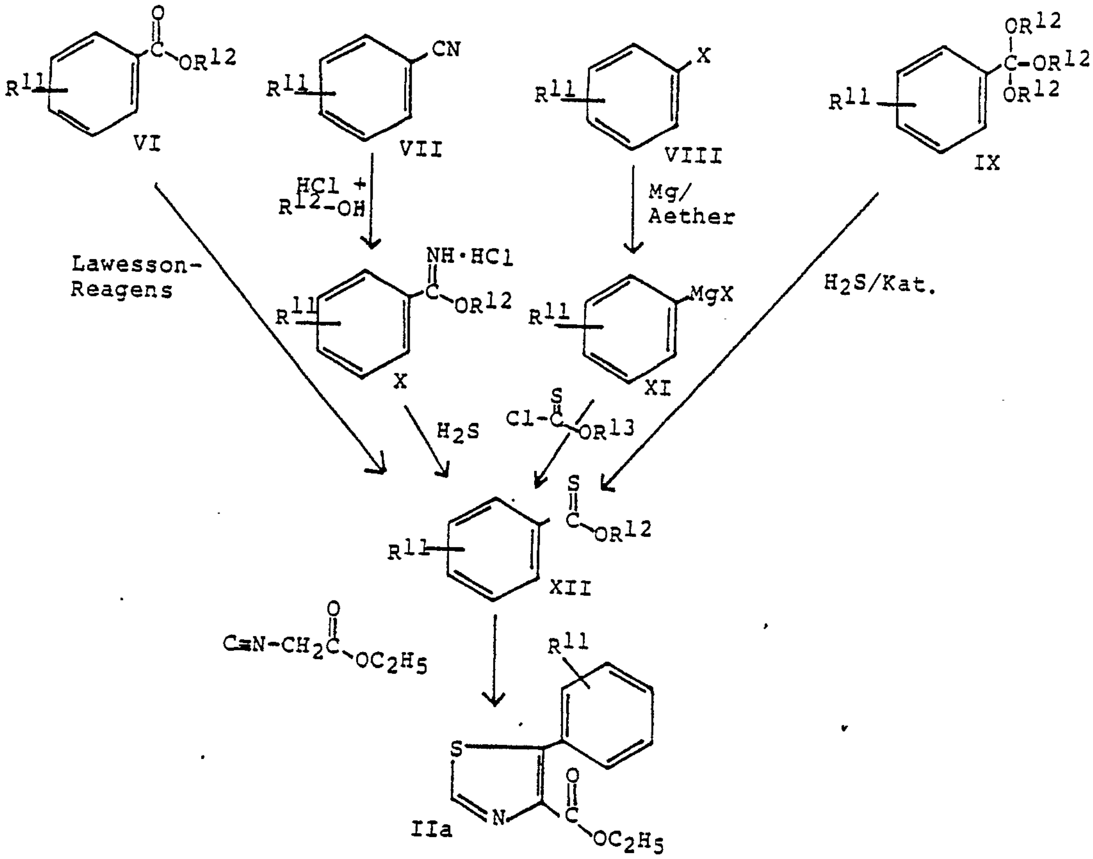


VI, VII, VIII, IX, X, XI, XII, IIa

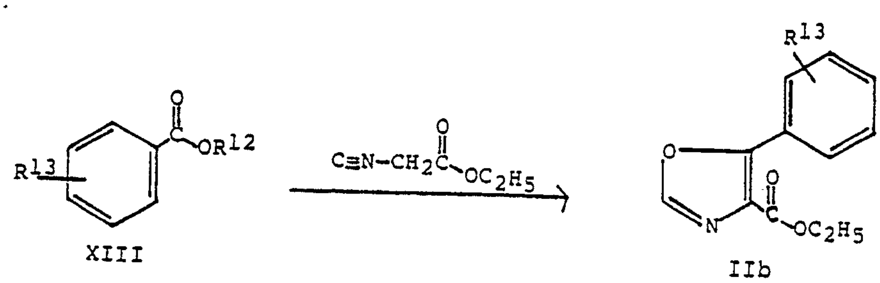

XIII, IIb

Schema II

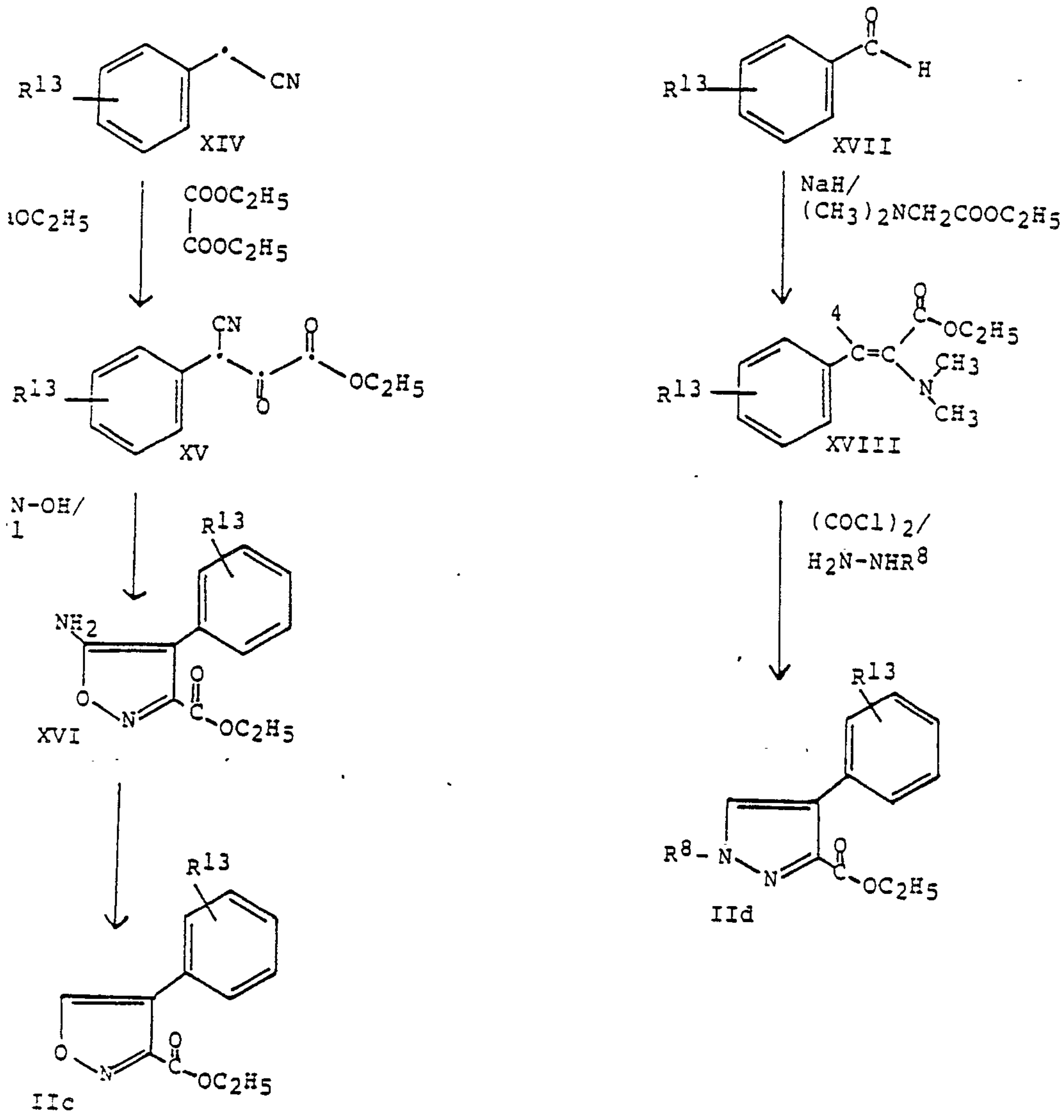


Die in Variante b) als Ausgangsstoffe verwendeten Verbindungen der Formel III sind ebenfalls neu, gehören aber wiederum bekannten Stoffklassen an und können in an sich bekannter Weise hergestellt werden. Sie bilden ebenfalls Gegenstand vorliegender Erfindung. Beispielsweise kann man eine Verbindung der Formel II oder ein reaktionsfähiges funktionelles Derivat davon unter den für Variante a) angegebenen Reaktionsbedingungen mit Aethanolamin umsetzen und das erhaltene N-(2-Hydroxyäthyl)carboxamid in an sich bekannter Weise, z.B. durch Umsetzen mit einem Halogenierungsmittel, wie Phosphortrichlorid, Phosphortribromid, Phosphorpentachlorid, Phosphoroxychlorid und dergleichen, einem Arylsulfonylhalogenid, wie Tosylchlorid, oder einem Alkylsulfonylhalogenid, wie Mesylchlorid, in die erwünschte Verbindung der Formel III überführen.

Die in Variante c) als Ausgangsstoffe verwendeten Verbindungen der Formel IV sind neu und ebenfalls Gegenstand der vorliegenden Erfindung. Sie können in an sich bekannter Weise hergestellt werden, beispielsweise, indem man eine Verbindung der Formel II oder ein reaktionsfähiges funktionelles Derivat davon unter den für Variante a) angegebenen Reaktionsbedingungen mit einer Verbindung der allgemeinen Formel

$H_2N-CH_2-CH_2-R^{10}$ V

worin $R^{10}$ obige Bedeutung hat, umsetzt. Die Verbindungen der Formel V sind bekannt oder können in Analogie zur Herstellung der bekannten Verbindungen erhalten werden.

Gemäss einem Alternativverfahren können die Verbindungen der Formel IV, worin $R^{10}$ Phthalimido, Azido oder Hexamethylentetrammonium bedeutet, auch durch Umsetzen einer Verbindung der Formel III mit Phthalimidkalium, einem Alkalimetallazid oder Hexamethylentetramin erhalten werden. Die Umsetzung erfolgt in sich bekannter Weise unter den für die Variante b) angegebenen Reaktionsbedingungen.

Die Verbindungen der Formel I und deren pharmazeutisch verwendbare Säureadditionssalze haben - wie bereits oben erwähnt - Monoaminoxydase (MAO) hemmende Aktivität. Auf Grund dieser Aktivität können die Verbindungen der Formel I und deren pharmazeutisch verwendbare Säureadditionssalze zur Behandlung von depressiven Zuständen und kognitiven Erkrankungen, insbesondere von solchen, die altersbedingt sind, verwendet werden. Beispiele solcher Erkrankungen sind altersbedingte Gedächtnisschwächen, primäre und sekundäre degenerative Demenz, z.B. Demenz von Alzheimer-Typ oder multiinfarktbedingte Demenz, und cerebrovaskuläre Erkrankungen und Folgen von Hirnschäden.

Die MAO hemmende Aktivität der erfindungsgemässen Verbindungen kann unter Verwendung von Standardmethoden bestimmt werden. So wurden die zu prüfenden Präparate dem nachfolgend beschriebenen in vitro-Test unterzogen, welcher sich an die von R.J. Wurtmann und J.A. Axelrod publizierte [Biochem. Pharmac. 12, 1439-1441 (1963)] Methode anlehnt.

Isolierte Rattengehirne werden im Verhältnis 1:4 (Gewicht/Volumen) in 0,1 molarem Kaliumphosphatpuffer (pH 7,4) homogenisiert, worauf die Homogenate im Verhältnis 1:4 (Volumen/Volumen) mit demselben Puffer verdünnt und bei -20° aufbewahrt werden. Zur Inkubation verwendet man eine mischung folgender Zusammensetzung:

- 100 µl 1 M Phosphatpuffer (pH 7,4);
- 100 µl Solubilisat der zu prüfenden Substanz in Wasser oder Dimethylsulfoxid;
- 20 µl Rattenhirnhomogenat; und als Substrat
- 80 µl $^{14}C$-Serotonin (5-HT) bzw. $^{14}C$-Phenyläthylamin (PEA), jeweils 100.000 Zerfälle pro Minute, entsprechend einer Endkonzentration von $2 \cdot 10^{-4}$ Mol/l bzw. $2 \cdot 10^{-5}$ Mol/l.

Vor Zugabe des Substrats erfolgt während 30 Minuten eine Vorinkubation bei 37° C. Die Inkubation (in Gegenwart des Substrats) erfolgt ebenfalls bei 37° C und dauert 10 Minuten. Die Reaktion wird durch Zugabe von 200 µl 2N Salzsäure gestoppt.

Zur Extraktion des deaminierten Produkts wird, je nach Verwendung von 5-HT bzw. von PEA als Substrat, während 10 Minuten mit 5 ml Diäthyläther bzw. mit 5 ml n-Heptan geschüttelt, worauf man zentrifugiert, die Wasserphase im Trockeneisbad ausfriert und die organische Phase in Zählgläser giesst.

Man bestimmt aufgrund der β-Zählerwerte die Aktivität der MAO im Vergleich zu Kontrollhomogenaten (ohne zu prüfende Substanz) und definiert als IC 50 diejenige Konzentration einer zu prüfenden Substanz, welche die Aktivität der MAO beim Substrat 5-HT bzw. PEA auf 50% herabsetzt.

Die so ermittelte Aktivität einiger erfindungsgemässer Verbindungen wird aus den in der folgenden Tabelle aufgeführten $IC_{50}$-Werten ersichtlich:

| Verbindung | IC 50, µMol/l | |
|---|---|---|
| "N-(2-Aminoäthyl)-...carboxamid" | 5-HT | PEA |
| ... 5-phenyl-4-thiazol ... | 0,02 | 20 |
| ... 5-(2-fluorphenyl)-4-thiazol... | 0,04 | >10 |
| ... 5-(3-fluorphenyl)-4-thiazol... | 0,02 | >10 |
| ... 5-(4-fluorphenyl)-4-thiazol... | 0,06 | 100 |
| ... 5-(4-chlorphenyl)-4-oxazol... | <0,01 | >100 |
| ... 5-(2-furyl)-4-oxazol... | 0,022 | 2,0 |
| ... 5-(3,5-dichlorphenyl)-4-thiazol... | 0,007 | 0,3 |
| ... 5-(2,4-difluorphenyl)-4-thiazol... | 0,12 | 3,6 |

Die Verbindungen der Formel I sowie deren pharmazeutisch verwendbare Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragées und Hartgelatinekapseln können die Verbindungen der Formel I und deren pharmazeutisch verwendbare Säureadditionssalze mit pharmazeutisch inerten, anorganischen oder organischen Excipientien verarbeitet werden. Als solche Excipientien kann man z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man Verbindungen der allgemeinen Formel I sowie deren pharmazeutisch verwendbare Säureadditionssalze bei der Bekämpfung bzw. Verhütung von depressiven Zuständen und kognitiven Erkrankungen, insbesondere von solchen, die altersbedingt sind, verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 50 bis 500 mg, vorzugsweise von etwa 100 bis 300 mg einer Verbindung der allgemeinen Formel I angemessen sein, wobei aber die soeben angegebene obere Grenze auch überschritten werden kann, wenn sich dies als angezeigt erweisen sollte.

Die folgenden Beispiele sollen die vorliegende Erfindung erläutern, sie jedoch in keiner Weise einschränken. Alle Temperaturen sind in Celsius Graden angegeben.

## Beispiel 1

(A) 4,3 g t-Butyl-[2-(5-phenyl-4-thiazolcarboxamido)äthyl]carbamat (12,37 mMol) werden in 10 ml Methylenchlorid gelöst, mit 5,0 ml Trifluoressigsäure versetzt und 16 Stunden bei 20° und 1 Stunde bei 60° gerührt. Anschliessend wird unter vermindertem Druck eingeengt. Der Rückstand wird in 10 ml Aethanol gelöst, mit 3 ml 17,5%iger (G/V) äthanolischer Salzsäure versetzt und eingedampft. Der Rückstand wird aus Methanol umkristallisiert, wobei man 3,3 g (94%) N-(2-Aminoäthyl)-5-phenyl-4-thiazolcarboxamid-hydrochlorid als weisse Kristalle erhält, Schmelzpunkt 243-245°.

Das als Ausgangsmaterial eingesetzte t-Butyl-[2-(5-phenyl-4-thiazolcarboxamido)äthyl]carbamat wurde wie folgt hergestellt:

(B) 70 ml (0,487 Mol) Benzoesäureäthylester werden mit 100 g (0,247 Mol) Lawesson-Reagens in 350 ml Xylol 12 Stun den zum Rückfluss erhitzt. Nach dem Abkühlen auf 20° wird das Reaktionsgemisch mit 500 ml Hexan verdünnt und abfiltriert. Das Filtrat wird an 1 kg Kieselgel flash-chromatographiert. Eluiert wird mit Hexan. Die nach dem Dünnschichtchromatogramm (Laufmittel: Toluol) reinen Fraktionen werden vereinigt und unter vermindertem Druck eingedampft. Man erhält 72 g (89%) Thiobenzoesäure-O-äthylester als dunkelgelbes übelriechendes Oel, welches ohne weitere Reinigung verwendet wird.

(C) Analog der in Synthesis 10, (1976), 681-682 beschriebenen Methode wird der Thiobenzoesäure-O-äthylester in Gegenwart von 1-5 % pulverisiertem Kaliumhydroxid in Aethanol mit Isocyanessigsäureäthylester in 70%iger Ausbeute zum 5-Phenyl-4-thiazolcarbonsäureäthylester umgesetzt, welcher nach Umkristallisation aus Essigester/Hexan bei 79-80° schmilzt.

(D) Der 5-Phenylthiazol-4-carbonsäureäthylester wird nach bekannten Methoden mit 2N wässriger Natriumhydroxidlösung 30 Minuten bei 70° hydrolysiert, wobei man nach Ansäuern die 5-Phenyl-4-thiazolcarbonsäure in 72%iger Ausbeute erhält, welche nach Umkristallisation aus Methanol/Diäthyläther bei 189-190° C schmilzt.

(E) Zu einer Lösung von 5,0 g (24,36 mMol) 5-Phenyl-4-thiazolcarbonsäure in 100 ml abs. Tetrahydrofuran werden 4,03 g (24,85 mMol) 1,1′-Carbonyldiimidazol gegeben, und das Reaktionsgemisch wird 2 Stunden bei 25° gerührt, wobei eine $CO_2$-Entwicklung stattfindet. Danach gibt man 4,1 g (25,59 mMol) t-Butyl-(2-aminoäthyl)carbamat zu. Man lässt das Reaktionsgemisch 2 Stunden bei 50° rühren und dampft es danach unter vermindertem Druck ein. Der ölige Rückstand wird in Methylenchlorid gelöst und an 50 g

Kieselgel chromatographiert. Eluiert wird zunächst mit Methylenchlorid, dann mit einem 8:2- und 7:3-Gemisch von Methylenchlorid und Essigester und schliesslich mit Essigester. Die nach dem Dünnschichtchromatogramm (Methylenchlorid/Essigester 7:3) reinen Fraktionen werden vereinigt und aus Essigester/Hexan umkristallisiert wobei man 7,7 g (91 %) t-Butyl-[2-(5-phenyl-4-thiazolcarboxamido)-äthyl]-carbamat als weisse Kristalle erhält, Schmelzpunkt 139-140°.

(E1) 4,8 g (20,58 mMol) 5-Phenyl-4-thiazolcarbonsäureäthylester und 4,0 g (25.0 mMol) t-Butyl-(2-aminoäthyl)carbamat werden unter vermindertem Druck 21 Stunden bei einer Badtemperatur von 100° erwärmt, wobei das gebildete Aethanol fortlaufend abdestilliert wird. Anschliessend wird abgekühlt, in 50 ml Methylenchorid gelöst und an 150 g Kieselgel chromatographiert. Eluiert wird zunächst mit Methylenchlorid, dann mit einem 9:1-, 8:2- und schliesslich 7:3-Gemisch von Methylenchlorid und Essigester. Die nach dem Dünnschichtchromatogramm reinen Fraktionen werden vereinigt und aus Essigester/Hexan umkristallisiert. Man erhält auf diese Weise 4,3 g (60,1 %) t-Butyl-[2-(5-phenyl-4-thiazolcarboxamide)äthyl]carbamat als weisse Kristalle, Schmelzpunkt 139-140°.

(E2) 2,0 g (9,75 mMol) 5-Phenyl-4-thiazolcarbonsäure werden mit 0,8 g (10,4 mMol) Chlorameisensäuremethylester in 20 ml Chloroform vorgelegt und auf 0° gekühlt. Dazu tropft man bei 0-5° 1,5 ml (10,8 mMol) Triäthylamin. Nach 30-minütigem Rühren bei 0-5° tropft man das Reaktionsgemisch innerhalb von 1,5 Stunden zu einer eisgekühlten Lösung von 1,6 g (10 mMol) t-Butyl-(2-aminoäthyl)carbamat in 20 ml Chloroform. Das Reaktionsgemisch wird noch 30 Minuten bei 0-5° gerührt, anschliessend mit 150 ml Methylenchlorid verdünnt und mit je 100 ml Wasser, gesättigter Natriumbicarbonatlösung sowie Natriumchloridlösung gewaschen. Die wässrigen Phasen werden mit 100 ml Methylenchlorid nachextrahiert. Die vereinigten organischen Phasen werden über Magnesiumsulfat getrocknet und eingedampft. Der kristalline Rückstand wird aus Essigester/Hexan umkristallisiert, wobei man nach dem Trocknen 2,4 g (70,9%) t-Butyl-[2-(5-phenyl-4-thiazolcarboxamido)äthyl]carbamat als weisse Kristalle erhält, Schmelzpunkt 139-140°.

## Beispiel 2

In analoger Weise wie in Beispiel 1(A) beschrieben wurden die folgenden Verbindungen hergestellt:

- aus t-Butyl-[2-[5-(2-fluorphenyl)-4-thiazolcarboxamido]äthyl]carbamat das N-(2-Aminoäthyl)-5-(2-fluorphenyl)-4-thiazolcarboxamid-hydrochlorid in 95%iger Ausbeute, Schmelzpunkt 239-240° (aus Methanol/Diäthyläther);
- aus t-Butyl-[2-[5-(3-fluorphenyl)-4-thiazolcarboxamido]äthyl]carbamat das N-(2-Aminoäthyl)-5-(3-fluorphenyl)-4-thiazolcarboxamid-hydrochlorid in 90%iger Ausbeute, Schmelzpunkt 244-246° (aus Methanol/Diäthyläther);
- aus t-Butyl-[2-[5-(4-fluorphenyl)-4-thiazolcarboxamido]äthyl]carbamat das N-(2-Aminoäthyl)-5-(4-fluorphenyl)-4-thiazolcarboxamid-hydrochlorid in 90%iger Ausbeute, Schmelzpunkt 258-259° (aus Methanol/Diäthyläther);
- aus t-Butyl-[2-[5-(2-chlorphenyl)-4-thiazolcarboxamido]äthyl]carbamat das N-(2-Aminoäthyl)-5-(2-chlorphenyl)-4-thiazolcarboxamid-hydrochlorid in 85%iger Ausbeute, Schmelzpunkt 264-266° (aus Methanol/Diäthyläther);
- aus t-Butyl-[2-[5-(3-chlorphenyl)-4-thiazolcarboxamido]äthyl]carbamat das N-(2-Aminoäthyl)-5-(3-chlorphenyl)-4-thiazolcarboxamid-hydrochlorid in g2%iger Ausbeute, Schmelzpunkt 257-258° (aus Methanol/Diäthyläther);
- aus t-Butyl-[2-[5-(4-chlorphenyl)-4-thiazolcarboxamido]äthyl]carbamat das N-(2-Aminoäthyl)-5-(4-chlorphenyl)-4-thiazolcarboxamid-hydrochlorid in 86%iger Ausbeute, Schmelzpunkt 272-274° (aus Methanol/Diäthyläther);
- aus t-Butyl-[2-[5-(3-bromphenyl)-4-thiazolcarboxamido]äthyl]carbamat das N-(2-Aminoäthyl)-5-(3-bromphenyl)-4-thiazolcarboxamid-hydrochlorid in 92%iger Ausbeute, Schmelzpunkt 260-261° (aus Methanol/Diäthyläther);
- aus t-Butyl-[2-[5-(3,5-dichlorphenyl)-4-thiazolcarboxamido]äthyl]carbamat das N-(2-Aminoäthyl)-5-(3,5-dichlorphenyl)-4-thiazolcarboxamid-hydrochlorid in 67%iger Ausbeute, Schmelzpunkt 294-295° (aus Methanol);
- aus t-Butyl-[2-[5-(2-methylphenyl)-4-thiazolcarboxamido]äthyl]carbamat das N-(2-Aminoäthyl)-5-(2-methylphenyl)-4-thiazolcarboxamid-hydrochlorid in 80%iger Ausbeute, Schmelzpunkt 278-279° (aus Methanol/Diäthyläther);
- aus t-Butyl-[2-[5-(3-methylphenyl)-4-thiazolcarboxamido]äthyl]carbamat das N-(2-Aminoäthyl)-5-(3-methylphenyl)-4-thiazolcarboxamid-hydrochlorid in 89%iger Ausbeute, Schmelzpunkt 261-262° (aus Methanol/Diäthyläther);

- aus t-Butyl-[2-[5-(4-methylphenyl)-4-thiazolcarboxamido]äthyl]carbamat das N-(2-Aminoäthyl)-5-(4-methylphenyl)-4-thiazolcarboxamid-hydrochlorid in 91%iger Ausbeute, Schmelzpunkt 259-261° (aus Methanol/Diäthyläther);
- aus t-Butyl-[2-[5-(2-methoxyphenyl)-4-thiazolcarboxamido]äthyl]carbamat das N-(2-Aminoäthyl)-5-(2-methoxyphenyl)-4-thiazolcarboxamid-hydrochlorid in 84%iger Ausbeute, Schmelzpunkt 230-231° (aus Methanol/Diäthyläther);
- aus t-Butyl-[2-[5-(3-methoxyphenyl)-4-thiazolcarboxamido]äthyl]carbamat das N-(2-Aminoäthyl)-5-(3-methoxyphenyl)-4-thiazolcarboxamid-hydrochlorid in 82%iger Ausbeute, Schmelzpunkt 242-243° (aus Methanol/Diäthyläther);
- aus t-Butyl-[2-[5-(4-methoxyphenyl)-4-thiazolcarboxamido]äthyl]carbamat das N-(2-Aminoäthyl)-5-(4-methoxyphenyl)-4-thiazolcarboxamid-hydrochlorid in 82%iger Ausbeute, Schmelzpunkt 263-265° (aus Methanol/Diäthyläther);
- aus t-Butyl-[2-[5-($\alpha$,$\alpha$,$\alpha$-trifluor-3-methylphenyl)-4-thiazolcarboxamido]äthyl]carbamat das N-(2-Aminoäthyl)-5-($\alpha$,$\alpha$,$\alpha$-trifluor-3-methylphenyl)-4-thiazolcarboxamid-hydrochlorid in 86%iger Ausbeute, Schmelzpunkt 226-227° (aus Methanol);
- aus t-Butyl-[2-[5-($\alpha$,$\alpha$,$\alpha$-trifluor-4-methylphenyl)-4-thiazolcarboxamido]äthyl]carbamat das N-(2-Aminoäthyl)-5-($\alpha$,$\alpha$,$\alpha$-trifluor-4-methylphenyl)-4-thiazolcarboxamid-hydrochlorid in 86%iger Ausbeute, Schmelzpunkt 281-284° (aus Methanol/Diäthyläther);
- aus t-Butyl-[2-[5-(3-cyanphenyl)-4-thiazolcarboxamido]äthyl]carbamat das N-(2-Aminoäthyl)-5-(3-cyanphenyl)-4-thiazolcarboxamid-hydrochlorid in 75%iger Ausbeute, Schmelzpunkt 244-246° (aus Methanol/Diäthyläther);
- aus t-Butyl-[2-[5-(4-cyanphenyl)-4-thiazolcarboxamido]äthyl]carbamat das N-(2-Aminoäthyl)-5-(4-cyanphenyl)-4-thiazolcarboxamid-hydrochlorid in 73%iger Ausbeute, Schmelzpunkt 250-252° (aus Methanol);
- aus t-Butyl-[2-[5-(2,4-dichlorphenyl)-4-thiazolcarboxamido]äthyl]carbamat das N-(2-Aminoäthyl)-5-(2,4-dichlorphenyl)-4-thiazolcarboxamid-hydrochlorid in 86%iger Ausbeute, Schmelzpunkt 278-280° (aus Methanol/Diäthyläther);
- aus t-Butyl-[2-[5-(3,4-dichlorphenyl)-4-thiazolcarboxamido]äthyl]carbamat das N-(2-Aminoäthyl)-5-(3,4-dichlorphenyl)-4-thiazolcarboxamid-hydrochlorid in 90%iger Ausbeute, Schmelzpunkt 244-247° (aus Methanol/Diäthyläther);
- aus t-Butyl-[2-[5-(2,4-difluorphenyl)-4-thiazolcarboxamido]äthyl]carbamat das N-(2-Aminoäthyl)-5-(2,4-difluorphenyl -4-thiazolcarboxamid-hydrochlorid in 94%iger Ausbeute, Schmelzpunkt 269-271° (aus Methanol);
- aus t-Butyl-[2-[5-(2-thienyl)-4-thiazolcarboxamido]äthyl]carbamat das N-(2-Aminoäthyl)-5-(2-thienyl)-4-thiazolcarboxamid-hydrochlorid in 93%iger Ausbeute, Schmelzpunkt 251-254° (aus Methanol/Diäthyläther);
- aus t-Butyl-[2-[5-(3-thienyl)-4-thiazolcarboxamido]äthyl]carbamat das N-(2-Aminoäthyl)-5-(3-thienyl)-4-thiazolcarboxamid-hydrochlorid in 89%iger Ausbeute, Schmelzpunkt 265-266° (aus Methanol/Diäthyläther);
- aus t-Butyl-[2-[5-(5-brom-2-thienyl)-4-thiazolcarboxamido]äthyl]carbamat das N-(2-Aminoäthyl)-5-(5-brom-2-thienyl)-4-thiazolcarboxamid-hydrochlorid in 77%iger Ausbeute, Schmelzpunkt 267-269° (aus Methanol/Diäthyläther);
- aus t-Butyl-[2-[5-(2-furyl)-4-thiazolcarboxamido]äthyl]carbamat das N-(2-Aminoäthyl)-5-(2-furyl)-4-thiazolcarboxamid-hydrochlorid in 80%iger Ausbeute, Schmelzpunkt 254° (aus Methanol/Diäthyläther);
- aus t-Butyl-[2-[5-(2,3-dichlorphenyl)-4-thiazolcarboxamido]äthyl]carbamat das N-(2-Aminoäthyl)-5-(2,3-dichlorphenyl)-4-thiazolcarboxamid-hydrochlorid in 96%iger Ausbeute, Schmelzpunkt 263-265° (aus Methanol/Diäthyläther);
- aus t-Butyl-[2-[5-(2,5-dichlorphenyl)-4-thiazolcarboxamido]äthyl]carbamat das N-(2-Aminoäthyl)-5-(2,5-dichlorphenyl)-4-thiazolcarboxamid-hydrochlorid in 80%iger Ausbeute, Schmelzpunkt 252-253° (aus Methanol); und
- aus t-Butyl-[2-[5-(3,4-difluorphenyl)-4-thiazolcarboxamido]äthyl]carbamat das N-(2-Aminoäthyl)-5-(3,4-difluorphenyl)-4-thiazolcarboxamid-hydrochlorid in 94%iger Ausbeute, Schmelzpunkt 242-244° (aus Methanol/Diäthyläther).

Die oben genannten, als Ausgangsstoffe eingesetzten Carbamate wurden wie folgt hergestellt:

In analoger Weise wie in Beispiel 1(E1) beschrieben wurden die folgenden Carbamate hergestellt:

- aus 5-(2-Fluorphenyl)-4-thiazolcarbonsäureäthylester und t-Butyl-(2-aminoäthyl)carbamat das t-Butyl-[2-[5-(2-fluorphenyl)-4-thiazolcarboxamido]äthyl]carbamat in 59%iger Ausbeute, Schmelzpunkt 143° (aus Essigester/Hexan);
- aus 5-(3-Fluorphenyl)-4-thiazolcarbonsäureäthylester und t-Butyl-(2-aminoäthyl)carbamat das t-Butyl-[2-[5-(3-fluorphenyl)-4-thiazolcarboxamido]äthyl]carbamat in 60%iger Ausbeute, Schmelzpunkt 142-143° (aus Essigester/Hexan);
- aus 5-(4-Fluorphenyl)-4-thiazolcarbonsäureäthylester und t-Butyl-(2-aminoäthyl)carbamat das t-Butyl-[2-[5-(4-fluorphenyl)-4-thiazolcarboxamido]äthyl]carbamat in 46%iger Ausbeute, Schmelzpunkt 110-111° (aus Essigester/Hexan);

- aus 5-(2-Chlorphenyl)-4-thiazolcarbonsäureäthylester und t-Butyl-(2-aminoäthyl)carbamat das t-Butyl-[2-[5-(2-chlorphenyl)-4-thiazolcarboxamido]äthyl]carbamat in 68%iger Ausbeute, Schmelzpunkt 166-167° (aus Essigester/Hexan);
- aus 5-(3-Chlorphenyl)-4-thiazolcarbonsäureäthylester und t-Butyl-(2-aminoäthyl)carbamat das t-Butyl-[2-[5-(3-chlorphenyl)-4-thiazolcarboxamido]äthyl]carbamat in 72%iger Ausbeute, Schmelzpunkt 122-123° (aus Essigester/Hexan);
- aus 5-(4-Chlorphenyl)-4-thiazolcarbonsäureäthylester und t-Butyl-(2-aminoäthyl)carbamat das t-Butyl-[2-[5-(4-chlorphenyl)-4-thiazolcarboxamido]äthyl]carbamat in 63%iger Ausbeute, Schmelzpunkt 147-148° (aus Essigester/Hexan);
- aus 5-(3-Bromphenyl)-4-thiazolcarbonsäureäthylester und t-Butyl-(2-aminoäthyl)carbamat das t-Butyl-[2-[5-(3-bromphenyl)-4-thiazolcarboxamido]äthyl]carbamat in 79%iger Ausbeute, Schmelzpunkt 124-125° (aus Essigester/Hexan);
- aus 5-(3,5-Dichlorphenyl)-4-thiazolcarbonsäureäthylester und t-Butyl-(2-aminoäthyl)carbamat das t-Butyl-[2-[5-(3,5-dichlorphenyl)-4-thiazolcarboxamido]äthyl]carbamat in 42%iger Ausbeute, Schmelzpunkt 130-132° (aus Essigester/Hexan);
- aus 5-(2-Methylphenyl)-4-thiazolcarbonsäureäthylester und t-Butyl-(2-aminoäthyl)carbamat das t-Butyl-[2-[5-(2-methylphenyl)-4-thiazolcarboxamido]äthyl]carbamat in 44%iger Ausbeute, Schmelzpunkt 163-164° (aus Essigester/Hexan);
- aus 5-(3-Methylphenyl)-4-thiazolcarbonsäureäthylester und t-Butyl-(2-aminoäthyl)carbamat das t-Butyl-[2-[5-(3-methylphenyl)-4-thiazolcarboxamido]äthyl]carbamat in 35%iger Ausbeute, Schmelzpunkt 112-113° (aus Essigester/Hexan);
- aus 5-(4-Methylphenyl)-4-thiazolcarbonsäureäthylester und t-Butyl-(2-aminoäthyl)carbamat das t-Butyl-[2-[5-(4-methylphenyl)-4-thiazolcarboxamido]äthyl]carbamat in 60%iger Ausbeute, Schmelzpunkt 133-134° (aus Essigester/Hexan);
- aus 5-(2-Methoxyphenyl)-4-thiazolcarbonsäureäthylester und t-Butyl-(2-aminoäthyl)carbamat das t-Butyl-[2-[5-(2-methoxyphenyl)-4-thiazolcarboxamido]äthyl]carbamat in 17,5%iger Ausbeute, Schmelzpunkt 142-143° (aus Essigester/Hexan);
- aus 5-(3-Methoxyphenyl)-4-thiazolcarbonsäureäthylester und t-Butyl-(2-aminoäthyl)carbamat das t-Butyl-[2-[5-(3-methoxyphenyl)-4-thiazolcarboxamido]äthyl]carbamat in 66%iger Ausbeute, Schmelzpunkt 98-100° (aus Essigester/Hexan);
- aus 5-(4-Methoxyphenyl)-4-thiazolcarbonsäureäthylester und t-Butyl-(2-aminoäthyl)carbamat das t-Butyl-[2-[5-(4-methoxyphenyl)-4-thiazolcarboxamido]äthyl]carbamat in 61%iger Ausbeute, Schmelzpunkt 102° (aus Essigester/Hexan);
- aus 5-(α,α,α-Trifluor-3-methylphenyl)-4-thiazolcarbonsäureäthylester und t-Butyl-(2-aminoäthyl)carbamat das t-Butyl-[2-[5-(α,α,α-trifluor-3-methylphenyl)-4-thiazolcarboxamido]äthyl]carbamat in 24%iger Ausbeute, Schmelzpunkt 119-120° (aus Essigester/Hexan);
- aus 5-(α,α,α-Trifluor-4-methylphenyl)-4-thiazolcarbonsäureäthylester und t-Butyl-(2-aminoäthyl)carbamat das t-Butyl-[2-[5-(α,α,α-trifluor-4-methylphenyl)-4-thiazolcarboxamido]äthyl]carbamat in 82%iger Ausbeute, Schmelzpunkt 152-153° (aus Essigester/Hexan);
- aus 5-(3-Cyanphenyl)-4-thiazolcarbonsäureäthylester und t-Butyl-(2-aminoäthyl)carbamat das t-Butyl-[2-[5-(3-cyanphenyl)-4-thiazolcarboxamido]äthyl]carbamat in 68%iger Ausbeute, Schmelzpunkt 127-128° (aus Essigester/Hexan);
- aus 5-(4-Cyanphenyl)-4-thiazolcarbonsäureäthylester und t-Butyl-(2-aminoäthyl)carbamat das t-Butyl-[2-[5-(4-cyanphenyl)-4-thiazolcarboxamido]äthyl]carbamat in 60%iger Ausbeute, Schmelzpunkt 149-149,5° (aus Essigester/Hexan);
- aus 5-(2-Thienyl)-4-thiazolcarbonsäureäthylester und t-Butyl-(2-aminoäthyl)carbamat das t-Butyl-[2-[5-(2-thienyl)-4-thiazolcarboxamido]äthyl]carbamat in 60%iger Ausbeute, Schmelzpunkt 100-101° (aus Essigester/Hexan);
- aus 5-(3-Thienyl)-4-thiazolcarbonsäureäthylester und t-Butyl-(2-aminoäthyl)carbamat das t-Butyl-[2-[5-(3-thienyl)-4-thiazolcarboxamido]äthyl]carbamat in 43%iger Ausbeute, Schmelzpunkt 83° (aus Aether);
- aus 5-(5-Brom-2-thienyl)-4-thiazolcarbonsäureäthylester und t-Butyl-(2-aminoäthyl)carbamat das t-Butyl-[2-[5-(5-brom-2-thienyl)-4-thiazolcarboxamido]äthyl]carbamat in 56%iger Ausbeute, Schmelzpunkt 144-145° (aus Essigester/Hexan) und
- aus 5-(2-Furyl)-4-thiazolcarbonsäureäthylester und t-Butyl-(2-aminoäthyl)carbamat das t-Butyl-[2-[5-(2-furyl)-4-thiazolcarboxamido]äthyl]carbamat in 56%iger Ausbeute, Schmelzpunkt 93-94° (aus Essigester/Hexan).

In analoger Weise wie in Beispiel 1(E) beschrieben wurden die folgenden Carbamate hergestellt:
- aus 5-(2,4-Dichlorphenyl)-4-thiazolcarbonsäure und t-Butyl-(2-aminoäthyl)carbamat das t-Butyl-[2-[5-(2,4-dichlorphenyl)-4-thiazolcarboxamido]äthyl]carbamat in 92,2%iger Ausbeute, Schmelzpunkt 142° (aus Essigester/Hexan);
- aus 5-(3,4-Dichlorphenyl)-4-thiazolcarbonsäure und t-Butyl-(2-aminoäthyl)carbamat das t-Butyl-[2-[5-(3,4-dichlorphenyl)-4-thiazolcarboxamido]äthyl]carbamat in 82,1%iger Ausbeute, Schmelzpunkt 128° (aus Essigester/Hexan);
- aus 5-(2,4-Difluorphenyl)-4-thiazolcarbonsäure und t-Butyl-(2-aminoäthyl)carbamat das t-Butyl-[2-[5-(2,4-difluorphenyl)-4-thiazolcarboxamido]äthyl]carbamat in 73%iger Ausbeute, Schmelzpunkt 109-110° (aus Essigester/Hexan);
- aus 5-(2,3-Dichlorphenyl)-4-thiazolcarbonsäure und t-Butyl-(2-aminoäthyl)carbamat das t-Butyl-[2-[5-(2,3-dichlorphenyl)-4-thiazolcarboxamido]äthyl]carbamat in 91%iger Ausbeute, Schmelzpunkt 144-145° (aus Essigester/Hexan);
- aus 5-(2,5-Dichlorphenyl)-4-thiazolcarbonsäure und t-Butyl-(2-aminoäthyl)carbamat das t-Butyl-[2-[5-(2,5-dichlorphenyl)-4-thiazolcarboxamido]äthyl]carbamat in 90%iger Ausbeute, Schmelzpunkt 129-130° (aus Essigester/Hexan); und
- aus 5-(3,4-Difluorphenyl)-4-thiazolcarbonsäure und t-Butyl-(2-aminoäthyl)carbamat das t-Butyl-[2-[5-(3,4-difluorphenyl)-4-thiazolcarboxamido]äthyl]carbamat in 93%iger Ausbeute, Schmelzpunkt 115-116° (aus Essigester/Hexan).

Die als Ausgangsstoffe eingesetzten 4-Thiazolcarbonsäureäthylester sind nachstehend aufgeführt. Sie wurden in analoger Weise wie in Beispiel 1 (C) beschrieben durch Umsetzen der entsprechenden Thioester mit Isocyanessigsäureäthylester in Gegenwart von Kaliumhydroxid hergestellt.
- aus 2-Fluorthiobenzoesäure-O-methylester der 5-(2-Fluorphenyl)-4-thiazolcarbonsäureäthylester in 67,3%iger Ausbeute als gelbes Oel;
- aus 3-Fluorthiobenzoesäure-O-methylester der 5-(3-Fluorphenyl)-4-thiazolcarbonsäureäthylester in 63,7%iger Ausbeute als hellgelbes Oel;
- aus 4-Fluorthiobenzoesäure-O-methylester der 5-(4-Fluorphenyl)-4-thiazolcarbonsäureäthylester in 51%iger Ausbeute, Schmelzpunkt 65-66° (aus Essigester/Hexan);
- aus 2-Chlorthiobenzoesäure-O-äthylester der 5-(2-Chlorphenyl)-4-thiazolcarbonsäureäthylester in 30%iger Ausbeute, als gelbes Oel;
- aus 3-Chlorthiobenzoesäure-O-äthylester der 5-(3-Chlorphenyl)-4-thiazolcarbonsäureäthylester in 41%iger Ausbeute, Schmelzpunkt 73-75° (aus Aether/Petroläther);
- aus 4-Chlorthiobenzoesäure-O-äthylester der 5-(4-Chlorphenyl)-4-thiazolcarbonsäureäthylester in 57%iger Ausbeute, Schmelzpunkt 99-100° (aus Essigester/Hexan);
- aus 3-Bromthiobenzoesäure-O-methylester der 5-(3-Bromphenyl)-4-thiazolcarbonsäureäthylester in 42%iger Ausbeute, Schmelzpunkt 66-67° (aus Essigester/Hexan);
- aus 3,5-Dichlorthiobenzoesäure-O-methylester der 5-(3,5-Dichlorphenyl)-4-thiazolcarbonsäureäthylester in 54%iger Ausbeute als Oel;
- aus 2-Methylthiobenzoesäure-O-methylester der 5-(2-Methylphenyl)-4-thiazolcarbonsäureäthylester in 23%iger Ausbeute, Schmelzpunkt 72-73° (aus Essigester/Hexan);
- aus 3-Methylthiobenzoesäure-O-methylester der 5-(3-Methylphenyl)-4-thiazolcarbonsäureäthylester in 37%iger Ausbeute als rotbraunes Oel;
- aus 4-Methylthiobenzoesäure-O-methylester der 5-(4-Methylphenyl)-4-thiazolcarbonsäureäthylester in 52%iger Ausbeute, Schmelzpunkt 78-79° (aus Essigester/Hexan);
- aus 2-Methoxythiobenzoesäure-O-methylester der 5-(2-Methoxyphenyl)-4-thiazolcarbonsäureäthylester in 8,6%iger Ausbeute als gelbes Oel;
- aus 3-Methoxythiobenzoesäure-O-methylester der 5-(3-Methoxyphenyl)-4-thiazolcarbonsäureäthylester in 25,6%iger Ausbeute als Oel;
- aus 4-Methoxythiobenzoesäure-O-methylester der 5-(4-Methoxyphenyl)-4-thiazolcarbonsäureäthylester in 48%iger Ausbeute, Schmelzpunkt 69-70° (aus Essigester/Hexan);
- aus 3-($\alpha,\alpha,\alpha$-Trifluormethyl)thiobenzoesäure-O-methylester der 5-($\alpha,\alpha,\alpha$-Trifluor-3-methylphenyl)-4-thiazolcarbonsäureäthylester in 51%iger Ausbeute als Oel;
- aus 4-($\alpha,\alpha,\alpha$-Trifluormethyl)thiobenzoesäure-O-methylester der 5-($\alpha,\alpha,\alpha$-Trifluor-4-methylphenyl)-4-thiazolcarbonsäureäthylester in 60%iger Ausbeute, Schmelzpunkt 124-125° (aus Essigester/Hexan);
- aus 3-Cyanthiobenzoesäure-O-methylester der 5-(3-Cyanphenyl)-4-thiazolcarbonsäureäthylester in 63%iger Ausbeute, Schmelzpunkt 149° (aus Essigester/Hexan);
- aus 4-Cyanthiobenzoesäure-O-methylester der 5-(4-Cyanphenyl)-4-thiazolcarbonsäureäthylester in 37,6%iger Ausbeute, Schmelzpunkt 111-112° (aus Essigester/Hexan);

- aus 2,4-Dichlorthiobenzoesäure-O-methylester der 5-(2,4-Dichlorphenyl)-4-thiazolcarbonsäureäthylester in 66,6%iger Ausbeute, Schmelzpunkt 79-80° (aus Essigester/Hexan);
- aus 3,4-Dichlorthiobenzoesäure-O-methylester der 5-(3,4-Dichlorphenyl)-4-thiazolcarbonsäureäthylester in 80,6%iger Ausbeute, Schmelzpunkt 113° (aus Essigester/Hexan);
- aus 2,4-Difluorthiobenzoesäure-O-methylester der 5-(2,4-Difluorphenyl)-4-thiazolcarbonsäureäthylester in 83%iger Ausbeute, Schmelzpunkt 73-74° (aus Essigester/Hexan);
- aus 2-Thiophenthiocarbonsäure-O-methylester der 5-(2-Thienyl)-4-thiazolcarbonsäureäthylester in 63%iger Ausbeute, Schmelzpunkt 37-38° (aus Essigester/Hexan);
- aus 3-Thiophenthiocarbonsäure-O-methylester der 5-(3-Thienyl)-4-thiazolcarbonsäureäthylester in 49%iger Ausbeute, Schmelzpunkt 65-67° (aus Essigester/Hexan);
- aus 5-Brom-2-thiophenthiocarbonsäure-O-methylester der 5-(5-Brom-2-thienyl)-4-thiazolcarbonsäureäthylester in 48%iger Ausbeute, Schmelzpunkt 80-86° (aus Essigester/Hexan);
- aus 2-Furanthiocarbonsäure-O-methylester der 5-(2-Furyl)-4-thiazolcarbonsäureäthylester in 70%iger Ausbeute, Schmelzpunkt 51-52° (aus Essigester/Hexan);
- aus 2,3-Dichlorthiobenzoesäure-O-methylester der 5-(2,3-Dichlorphenyl)-4-thiazolcarbonsäureäthylester in 83%iger Ausbeute, Schmelzpunkt 131° (aus Aethanol);
- aus 2,5-Dichlorthiobenzoesäure-O-methylester der 5-(2,5-Dichlorphenyl)-4-thiazolcarbonsäureäthylester in 56%iger Ausbeute, Schmelzpunkt 89-90° (aus Essigester/Hexan) und
- aus 3,4-Difluorthiobenzoesäure-O-methylester der 5-(3,4-Difluorphenyl)-4-thiazolcarbonsäureäthylester in 78%iger Ausbeute, Schmelzpunkt 75° (aus Essigester/Hexan).

Die als Ausgangstoffe eingesetzten 4-Thiazolcarbonsäuren wurden durch Hydrolyse in analoger Weise wie in Beispiel 1(D) beschrieben aus den entsprechenden 4-Thiazolcarbonsäuremethylestern hergestellt:

Die 5-(2,4-Dichlorphenyl)-4-thiazolcarbonsäure in 78%iger Ausbeute, Schmelzpunkt 185-186° (aus Essigester/Hexan);
die 5-(3,4-Dichlorphenyl)-4-thiazolcarbonsäure in 95%iger Ausbeute, Schmelzpunkt 202-203° (aus Wasser);
die 5-(2,4-Difluorphenyl)-4-thiazolcarbonsäure in 95%iger Ausbeute, Schmelzpunkt 182-183° (aus Wasser);
die 5-(2,3-Dichlorphenyl)-4-thiazolcarbonsäure in 97%iger Ausbeute, Schmelzpunkt 199-201° (aus Wasser);
die 5-(2,5-Dichlorphenyl)-4-thiazolcarbonsäure in 95%iger Ausbeute, Schmelzpunkt 172-173° (aus Wasser); und
die 5-(3,4-Difluorphenyl)-4-thiazolcarbonsäure in 55%iger Ausbeute, Schmelzpunkt 195-196° (aus Wasser).

Die oben als Ausgangstoffe eingesetzten Thioester schliesslich wurden aus den entsprechenden Carbonsäureestern durch Umsetzen mit Lawesson-Reagens in analoger Weise wie in Beispiel 1(B) beschrieben erhalten:

Der 2-Fluorthiobenzoesäure-O-methylester in 51%iger Ausbeute als gelbes Oel;
der 3-Fluorthiobenzoesäure-O-methylester in 53%iger Ausbeute als gelbes Oel;
der 4-Fluorthiobenzoesäure-O-methylester in 91%iger Ausbeute als gelbes Oel;
der 2-Chlorthiobenzoesäure-O-äthylester in 62%iger Ausbeute als gelbes Oel;
der 3-Chlorthiobenzoesäure-O-äthylester in 80%iger Ausbeute als gelbes Oel;
der 4-Chlorthiobenzoesäure-O-äthylester in 70%iger Ausbeute als oranges Oel;
der 3-Bromthiobenzoesäure-O-äthylester in 43%iger Ausbeute als gelbes Oel;
der 3,5-Dichlorthiobenzoesäure-O-methylester in 41%iger Ausbeute als gelbes Oel;
der 2-Methylthiobenzoesäure-O-methylester in 74%iger Ausbeute als gelbes Oel;
der 3-Methylthiobenzoesäure-O-methylester in 76%iger Ausbeute als dunkelgelbes Oel;
der 4-Methylthiobenzoesäure-O-methylester in 75%iger Ausbeute als gelbes Oel;
der 2-Methoxythiobenzoesäure-O-methylester in 50%iger Ausbeute als oranges Oel;
der 3-Methoxythiobenzoesäure-O-methylester in 72,5%iger Ausbeute als oranges Oel;
der 4-Methoxythiobenzoesäure-O-methylester in 52%iger Ausbeute als oranges Oel;
der 3-($\alpha,\alpha,\alpha$-Trifluormethyl)thiobenzoesäure-O-methylester in 59%iger Ausbeute als gelbes Oel;
der 4-($\alpha,\alpha,\alpha$-Trifluormethyl)thiobenzoesäure-O-methylester in 45%iger Ausbeute als gelbes Oel;
der 3-Cyanthiobenzoesäure -O-methylester in 43%iger Ausbeute als gelbes Oel;
der 4-Cyanthiobenzoesäure-O-methylester in 88%iger Ausbeute, Schmelzpunkt 53-54°;
der 2,4-Dichlorthiobenzoesäure-O-methylester in 20%iger Ausbeute als gelbes Oel;
der 3,4-Dichlorthiobenzoesäure-O-methylester in 50%iger Ausbeute als gelbes Oel;
der 2,4-Difluorthiobenzoesäure-O-methylester in 69,4%iger Ausbeute als gelbes Oel;
der 2-Thiophenthiocarbonsäure-O-methylester in 93%iger Ausbeute als gelbes Oel;
der 3-Thiophenthiocarbonsäure-O-methylester in 81%iger Ausbeute als gelbes Oel;
der 5-Brom-2-thiophenthiocarbonsäure-O-methylester in 79%iger Ausbeute als gelbes Oel;
der 2-Furanthiocarbonsäure-O-methylester in 72,4%iger Ausbeute als oranges Oel;

der 2,3-Dichlorthiobenzoesäure-O-methylester in 36%iger Ausbeute als gelbes Oel;
der 2,5-Dichlorthiobenzoesäure-O-methylester in 26%iger Ausbeute als gelbes Oel und
der 3,4-Difluorthiobenzoesäure-O-methylester in 67%iger Ausbeute als gelbes Oel.

## Beispiel 3

In analoger Weise wie Beispiel 1(A) beschrieben wurden aus 19,4 g (42,8 mMol) t-Butyl-[2-[5-[3-(benzyloxy)phenyl]-4-thiazolcarboxamido]äthyl]carbamat 14,7 g (91%) N-(2-Aminoäthyl)-5-[3-(benzyloxy)-phenyl]-4-thiazolcarboxamid-hydrochlorid als beige Kristalle erhalten, Schmelzpunkt 230-231° (aus Methanol).

Das als Ausgangsmaterial eingesetzte t-Butyl-[2-[5-[3-(benzyloxy)phenyl]-4-thiazolcarboxamido]äthyl]-carbamat wurde wie folgt hergestellt:

40 g (0,165 Mol) 3-(Benzyloxy)benzoesäuremethylester werden in analoger Weise wie in Beispiel 1(B) beschrieben mit 33,4 g (0.0826 Mol) Lawesson-Reagens in Xylol 8 Stunden am Rückfluss erhitzt und danach an Kieselgel chromatographiert, wobei 21,7 g (51%) 3-(Benzyloxy)thiobenzoesäure-O-methylester als rotbraunes Oel erhalten werden, welches beim stehen lassen allmählich kristallisiert.

In analoger Weise wie in Beispiel 1(C) beschrieben werden 21,7 g 3-(Benzyloxy)thiobenzoesäure-O-methylester (44,13 mMol) mit Isocyanessigsäureäthylester umgesetzt, wobei man 26,5 g (92,9%) 5-[3-(Benzyloxy)phenyl]-4-thiazolcarbonsäureäthylester als oranges Oel erhält.

Aus 26,5 g (78,1 mMol) 5-[3-(Benzyloxy)phenyl]-4-thiazolcarbonsäureäthylester erhält man durch Umsetzen mit t-Butyl-(2-aminoäthyl)carbamat 19,4 g (54,8%) t-Butyl-[2-[5-[3-(benzyloxy)phenyl]-4-thiazolcarboxamido]äthyl]carbamat als Oel, welches ohne weitere Reinigung verwendet wird.

## Beispiel 4

Aus 8,4 g (17,2 mMol) t-Butyl-[2-[5-[3-[(3-chlorbenzyl)oxy]phenyl]-4-thiazolcarboxamido]äthyl]carbamat wurden in analoger Weise wie in Beispiel 1(A) beschrieben 6,4 g N-(2-Aminoäthyl)-5-[3-[(3-chlorbenzyl)oxy]-phenyl]-4-thiazolcarboxamid-hydrochlorid als weisse Kristalle erhalten, Schmelzpunkt 212-213° (aus Methanol/Aether, Ausbeute: 87,6%).

Das als Ausgangsmaterial eingesetzte t-Butyl-[2-[5-[3-[(3-chlorbenzyl)oxy]phenyl]-4-thiazolcarboxamido)äthyl] carbamat wurde wie folgt hergestellt:

In analoger Weise wie in Beispiel 1(B) beschrieben wurden 36,5 g (131,9 mMol) 3-[(3-Chlorbenzyl)oxy]-benzoesäuremethylester mit 26,7 g (66 mMol) Lawesson-Reagens in Xylol 16 Stunden am Rückfluss erhitzt und danach an Kieselgel chromatographiert. Eluieren mit Hexan/Toluol (2%, 5% und 10%) lieferte 28,2 g (73%) 3-[(3-Chlorbenzyl)oxy]thiobenzoesäure-O-methylester als oranges, kristallisierendes Oel.

In analoger Weise wie in Beispiel 1(C) beschrieben wurden 28,2 g (96,3 mMol) 3-[(3-Chlorbenzyl)oxy]-thiobenzoesäure-O-methylester mit Isocyanessigsäureäthylester umgesetzt und anschliessend an Kieselgel chromatographiert. Eluieren mit 9:1-, 8:2- und 1:1-Gemischen von Methylenchlorid und Essigester sowie Essigester allein lieferte 28 g (77,8%) 5-[3-[(3-Chlorbenzyl)oxy]phenyl]-4-thiazolcarbonsäureäthylester als oranges Oel.

Aus 10,0 g (26,75 mMol) 5-[3-[(3-Chlorbenzyl)oxy]phenyl]-4-thiazolcarbonsäureäthylester wurden durch Umsetzen mit t-Butyl-(2-aminoäthyl)carbamat 10,5 g Kristalle erhalten, welche aus Essigester/Hexan umkristallisiert wurden. Man erhielt auf diese Weise 8,4 g (64,4%) t-Butyl-[2-[5-[3-[(3-chlorbenzyl)oxy]phenyl]-4-thiazolcarboxamido]äthyl]carbamat als weisse Kristalle, Schmelzpunkt 116-117°.

## Beispiel 5

Aus 2,9 g (6.06 mMol) t-Butyl-[2-(5-(3-[(3-cyanbenzyl)oxy]phenyl)-4-thiazolcarboxamido)äthyl]carbamat wurden in analoger Weise wie in Beispiel 1 (A) beschrieben 2,1 g (83,5%) N-(2-Aminoäthyl)-5-(3-[(3-cyanbenzyl)oxy]phenyl)-4-thiazolcarboxamid-hydrochlorid als weisse Kristalle erhalten, Schmelzpunkt 211-215 (aus Methanol/Aether).

Das als Ausgangsmaterial eingesetzte t-Butyl-[2-(5-(3-[(3-cyanbenzyl)oxy]phenyl)-4-thiazolcarboxamido)-

äthyl]carbamat wurde wie folgt hergestellt:

8,9 g (33,3 mMol) 3-[(3-Cyanbenzyl)oxy]benzoesäuremethylester wurden in analoger Weise wie in Beispiel 1 (B) beschrieben mit 13,5 g (33,4 mMol) Lawesson-Reagens in Xylol 17 Stunden zum Rückfluss erhitzt und danach an Kieselgel chromatographiert. Eluieren mit einem 8:2- und 7:3-Gemisch von Hexan und Methylenchlorid lieferte 7,6 g (65,8%) 3-[(3-Cyanbenzyl)oxy]thiobenzoesäure-O-methylester als gelbes Oel, welches beim Stehenlassen allmählich kristallisiert.

7,6 g (26,82 mMol) 3-[(3-Cyanbenzyl)oxy]thiobenzoesäure-O-methylester werden zusammen mit 3,6 g (32,1 mMol) Isocyanessigsäureäthylester in 50 ml Aethanol gelöst und bei Raumtemperatur zu einer Lösung von 0,4 g Kaliumhydroxyd in 30 ml Aethanol getropft. Nach beendeter Zugabe wird das Reaktionsgemisch noch 20 Stunden unter Rühren zum Rückfluss erhitzt und dann eingedampft. Der Rückstand wird zwischen Aether und Wasser verteilt, die wässrige Phase wird noch 2 mal mit Aether extrahiert, und die vereinigten organischen Phasen werden mit Wasser und gesättigter Kochsalzlösung gewaschen, über Magnesiumsulfat getrocknet, filtriert und eingedampft. Der Rückstand wird an 150 g Kieselgel mit Methylenchlorid und Methylenchlorid, welches 5% bzw. 10% Essigester enthält, als Eluierungsmittel chromatographiert. Die reinen Fraktionen werden vereinigt und eingedampft, wobei man 4,5 g eines Oel erhält, welches aus Essigester/Hexan kristallisiert. Auf diese Weise werden 3,5 g (36%) 5-[3-[(3-Cyanbenzyl)oxy]phenyl]-4-thiazolcarbonsäureäthylester als beige Kristalle erhalten, Schmelzpunkt 85°.

Aus 3,5 g (9,6 mMol) 5-[3-[(3-Cyanbenzyl)oxy]phenyl]-4-thiazolcarbonsäureäthylester wurden durch Umsetzen mit t-Butyl-(2-aminoäthyl)carbamat in analoger Weise wie in Beispiel 1 (E1) beschrieben und anschliessende Chromatographie an Kieselgel mit einem 9:1- und 8:2-Gemisch von Methylenchlorid und Essigester als Eluierungsmittel 3,4 g eines Oels erhalten, welches aus Essigester/Hexan kristallisierte. Auf diese Weise wurden 3,0 g (65,3%) t-Butyl-[2-(5-[3-[(3-cyanbenzyl)oxy]phenyl)-4-thiazolcarboxamido)äthyl]-carbamat als weisse Kristalle erhalten, Schmelzpunkt 115°.

## Beispiel 6

2,0 g (5,5 mMol) t-Butyl-[2-(2-amino-5-phenyl-4-thiazolcarboxamido)äthyl]carbamat werden in 15 ml Methylenchlorid gelöst und mit 2,1 ml (27,6 mMol) Trifluoressigsäure 3 Stunden zum Rückfluss erhitzt. Danach wird das Reaktionsgemisch unter vermindetem Druck eingedampft, und der Rückstand wird in Methanol gelöst, mit ~ 2 M methanolischer Salzsäure versetzt und eingedampft. Umkristallisation des Rückstands aus Methanol/Aether unter Zusatz von wenig Hexan liefert 1,65 g (90%) 2-Amino-N-(2-aminoäthyl)- 5-phenyl-4-thiazolcarboxamid-dihydrochlorid als weisse Kristalle, Schmelzpunkt 203-205°.

In analoger Weise wie oben beschrieben wurden die beiden folgenden Verbindungen hergestellt:

- aus 0,9 g (2,27 mMol) t-Butyl-[2-(2-amino-5-(4-chlorphenyl)-4-thiazolcarboxamido)äthyl]carbamat 0,8 g (95%) 2-Amino-N-(2-aminoäthyl)-5-(4-chlorphenyl)-4-thiazolcarboxamid-dihydrochlorid als weisse Kristalle, Schmelzpunkt 228-230° und
- aus 2,1 g (5,52 mMol) t-Butyl-[2-(2-amino-5-(3-fluorphenyl)-4-thiazolcarboxamido)äthyl]carbamat 0,7 g (36%) 2-Amino-N-(2-aminoäthyl)-5-(3-fluorphenyl)-4-thiazolcarboxamid-hydrochlorid als weisse Kristalle, Schmelzpunkt 296-297°.

Das als Ausgangsmaterial eingesetzte t-Butyl-[2-(2-amino-5-phenyl)-4-thiazolcarboxamido)äthyl]-carbamat wurde wie folgt hergestellt:

5,0 g (21,3 mMol) 2-Amino-5-phenyl-4-thiazolcarbonsäuremethylester (welcher wie in J.Chem.Soc.Perk.Trans.I (1982) 159-164 beschrieben hergestellt wurde) und 6,84 g (42,7 mMol) t-Butyl-(2-aminoäthyl)carbamat werden unter vermindertem Druck 2 Stunden bei einer Badtemperatur von 140° erwärmt, wobei das gebildete Methanol laufend abdestilliert wird. Nach dem Abkühlen wird der Rückstand in 50 ml Methylenchlorid gelöst und an 200 g Kieselgel mit einem 9:1-Gemisch von Methylenchlorid und Methanol als Eluierungsmittel chromatographiert. Die nach dem Dünnschichtchromatogramm reinen Fraktionen werden vereinigt und unter vermindertem Druck eingedampft, wobei man 4,5 g eines roten, kristallisierenden Oels erhält. Kristallisation aus Methanol und einem Aether/Hexangemisch liefert 3,48 g (45%) t-Butyl-[2-(2-amino-5-phenyl-4-thiazolcarboxamido)äthyl]carbamat als beige Kristalle, welche ohne weitere Reinigung verwendet werden.

In analoger Weise wie oben beschrieben wurden die folgenden beiden Carbamate hergestellt:

- aus 4,0 g (14,89 mMol) 2-Amino-5-(4-chlorphenyl)-4-thiazolcarbonsäuremethylester 1,2 g(20%) t-Butyl-[2-(2-amino-5-(4-chlorphenyl)-4-thiazolcarboxamide)äthyl]carbamat als fast weisse Kristalle, Schmelzpunkt 156-157° und
- aus 5,0 g (19,82 mMol) 2-Amino-5-(3-fluorphenyl)-4-thiazolcarbonsäuremethylester 2,1 g (27,5%) t-Butyl-[2-(2-amino-5-(3-fluorphenyl)-4-thiazolcarboxamido)äthyl]carbamat als weisse Kristalle, Schmelzpunkt 178-179°.

Die beiden als Ausgangsstoffe eingesetzten Methylester wurden nach der in J.Chem.Soc.Perk.Trans. I (1982) 159-164 beschriebenen Methode hergestellt:

- 2-Amino-5-(4-chlorphenyl)-4-thiazolcarbonsäuremethylester in 95%iger Ausbeute, Schmelzpunkt 242-244° (aus Wasser) und
- 2-Amino-5-(3-fluorphenyl)-4-thiazolcarbonsäuremethylester in 86,9%iger Ausbeute, Schmelzpunkt 224-226°.

## Beispiel 7

In analoger Weise wie in Beispiel 6 beschrieben wurden aus 1,55 g (4,1 mMol) t-Butyl-[2-(2-chlor-5-phenyl-4-thiazolcarboxamido)äthyl]carbamat 1,23 g (94%) N-(2-Aminoäthyl)-2-chlor-5-phenyl-4-thiazolcarboxamid-hydrochlorid als weisse Kristalle erhalten, Schmelzpunkt 186-187°.

Das als Ausgangsmaterial eingesetze t-Butyl-[2-(2-chlor-5-phenyl-4-thiazolcarboxamido)äthyl]carbamat wurde wie folgt hergestellt:

14,04 g (60 mMol) 2-Amino-5-phenyl-4-thiazolcarbonsäuremethylester werden wie in J. Chem. Soc. Perk. Trans. I (1982), S. 159-164 beschrieben umgesetzt; man erhält ca. 15 g eines gelben Oels. Chromatographie an Kieselgel mit Methylenchlorid als Eluierungsmittel und Kristallisation des erhaltenen Rohprodukts aus Aether/Hexan liefert 10,6 g (70%) 2-Chlor-5-phenyl-4-thiazolcarbonsäuremethylester als Kristalle, Schmelzpunkt 68-69°.

5,4 g (21,3 mMol) 2-Chlor-5-phenyl-4-thiazolcarbonsäuremethylester und 3,6 g (22,3 mMol) t-Butyl-(2-aminoäthyl)carbamat werden unter vermindertem Druck 22 Stunden bei einer Badtemperatur von 110° gerührt, wobei das gebildete Methanol laufend abdestilliert wird. Das Reaktionsgemisch wird abgekühlt, in Methylenchlorid gelöst und an 150 g Kieselgel mit Methylenchlorid und einem 4:1-Gemisch von Methylenchorid und Essigester als Eluierungsmittel chromatographiert. Die nach dem Dünnschichtchromatogramm reinen Fraktionen werden vereinigt und eingedampft. Kristallisation des Rückstands aus Essigester/Hexan liefert 1,83 g (27,5%) t-Butyl-[2-(2-chlor-5-phenyl-4-thiazolcarboxamido)äthyl]carbamat als weisse Kristalle, Schmelzpunkt 114-116°.

## Beispiel 8

In analoger Weise wie in Beispiel 6 beschrieben wurden aus 1,2 g (3,0 mMol) t-Butyl-[2-(2-chlor-5-(3-fluorphenyl)-4-thiazolcarboxamido)äthyl]carbamat 0,75 g (76%) N-(2-Aminoäthyl)-2-chlor-5-(3-fluorphenyl)-4-thiazolcarboxamidhydrochlorid als weisse Kristalle erhalten, Schmelzpunkt 206-207°.

Das als Ausgangsmaterial eingesetze t-Butyl-[2-(2-chlor-5-(3-fluorphenyl)-4-thiazolcarboxamido)äthyl]-carbamat wurde wie folgt hergestellt:

In analoger Weise wie in Beispiel 7 beschrieben wurden aus 5,0 g (19,82 mMol) 2-Amino-5-(3-fluorphenyl)-4-thiazolcarbonsäuremethylester 2,0 g (37,1%) 2-Chlor-5-(3-fluorphenyl)-4-thiazolcarbonsäuremethylester als weisse Kristalle erhalten, Schmelzpunkt 99-100° (aus Essigester/Hexan).

In analoger Weise wie in Beispiel 7 beschrieben wurden aus 2,6 g (9,57 mMol) 2-Chlor-5-(3-fluorphenyl)-4-thiazolcarbonsäuremethylester 1,2 g (32%) t-Butyl-[2-(2-chlor-5-(3-fluorphenyl)-4-thiazolcarboxamido)äthyl]carbamat als weisse Kristalle erhalten, welche direkt weiter verwendet wurden.

## Beispiel 9

0,5 g (1,2 mMol) t-Butyl-[2-(2-brom-5-phenyl-4-thiazolcarboxamido)äthyl]carbamat, 2 ml Trifluoressigsäure und 10 ml Methylenchlorid werden 2 Stunden zum Rückfluss erhitzt und dann eingedampft. Der Rückstand wird in Methanol gelöst und mit 1 ml Bromwasserstoffsäure in Eisessig (~ 30%) versetzt. Erneutes Eindampfen und Umkristallisieren des Rückstands aus Methanol/Aether liefert 0,45 g (94,2%) N-(2-Aminoäthyl)-2-brom-5-phenyl-4-thiazolcarboxamid-hydrobromid als beige Kristalle, Schmelzpunkt 214-215° (Zers.).

Das als Ausgangsmaterial eingesetze t-Butyl-[2-(2-brom-5-phenyl-4-thiazolcarboxamido)äthyl]carbamat wurde wie folgt hergestellt:

Aus 7.02 g (30 mMol) 2-Amino-5-phenyl-4-thiazolcarbonsäuremethylester, 15,48 g (62 mMol) Kupfersulfat, 13,73 g (133 mMol) Natriumbromid, 5,1 g (73,9 mMol) Natriumnitrit und 300 g konz. Schwefelsäure in 225 ml Wasser wurden in analoger Weise wie in Beispiel 7 beschrieben 7,0 g (78%) 2-Brom-5-phenyl-4-thiazolcarbonsäuremethylester als Kristalle erhalten, welche nach Umkristallisieren aus Aether/Hexan bei 86-87° schmelzen.

2,0 g (6,7 mMol) 2-Brom-5-phenyl-4-thiazolcarbonsäuremethylester und 1,61 g (10,1 mMol) t-Butyl-(2-aminoäthyl)carbamat werden unter vermindertem Druck 2,5 Stunden bei 110° Badtemperatur gerührt, wobei das gebildete Methanol laufend abdestilliert wird. Nach Chromatographieren an 100 g Kieselgel mit Methylenchlorid und 4:1-Gemisch von Methylenchlorid und Essigester erhält man 0,58 g (26%) t-Butyl-[2-(2-brom-5-phenyl-4-thiazolcarboxamido)äthyl]carbamat als hellgelbes, kristallisierendes Oel, welches direkt weiter verwendet wird.

## Beispiel 10

Zu einer Suspension von 3,1 g (19,1 mMol) 1,1′-Carbonyldiimidazol in 20 ml trockenem Tetrahydrofuran werden portionsweise 3,6 g (19,1 mMol) 5-Phenyl-4-oxazolcarbonsäure gegeben, wobei sich unter Gasentwicklung eine braune Lösung bildet. Nach einer Reaktionszeit von 15 Minuten bei 20° werden 3,05 g (19,0 mMol) t-Butyl-(2-aminoäthyl)carbamat in 10 ml trockenem Tetrahydrofuran zugetropft (leichte Erwärmung) und das Reaktionsgemisch 30 Minuten weitergerührt. Danach dampft man das Reaktionsgemisch unter vermindertem Druck ein und verteilt den Rückstand zwischen Methylenchlorid und 0,1 N-Salzsäure. Nach dem Neutralwaschen mit gesättigter Kochsalzlösung wird die organische Phase über Magnesiumsulfat getrocknet und eingedampft, wobei 5,8 g (92%) t-Butyl-[2-(5-phenyl-4-oxazolcarboxamido)äthyl]carbamat erhalten werden, welche in 20 ml Trifluoressigsäure 20 Minuten bei 20° gerührt werden. Danach wird das Reaktionsgemisch unter vermindertem Druck eingedampft. Der Rückstand wird in 5 ml Methanol gelöst und mit 10 ml 5,5 N Chlorwasserstoff in Methanol versetzt. Abfiltrieren der ausgefallenen Kristalle liefert 2,4 g (51%) N-(2-Aminoäthyl)-5-phenyl-4-oxazolcarboxamid-hydrochlorid als weisse Kristalle, Schmelzpunkt 257° (Zers.).

Die als Ausgangsmaterial eingesetzte 5-Phenyl-4-oxazolcarbonsäure wurde wie folgt hergestellt:

Analog der in Tetrahedron Letters, 23, 235-236 (1982) beschriebenen Methode wurde aus Isocyanessigsäureäthylester, Benzoesäure, Phosphorsäure-diphenylesterazid und Kaliumcarbonat in Dimethylformamid/Wasser der 5-Phenyl-4-oxazolcarbonsäureäthylester hergestellt, welcher dann ebenfalls nach bekannten Methoden durch Hydrolyse mit 2 N Natronlauge bei 50° während 15 Minuten in die erwünschte Säure übergeführt wurde.

## Beispiel 11

In analoger Weise wie in Beispiel 10 beschrieben wurden die folgenden Verbindungen hergestellt:

- aus t-Butyl-[2-[5-(2-chlorphenyl-4-oxazolcarboxamido]äthyl]carbamat das N-(2-Aminoäthyl)-5-(2-chlorphenyl)-4-oxazolcarboxamid-hydrochlorid in 66,4%iger Ausbeute, Schmelzpunkt 247-248° (aus Methanol/Diäthyläther);
- aus t-Butyl-[2-[5-(3-chlorphenyl)-4-oxazolcarboxamido]äthyl]carbamat das N-(2-Aminoäthyl)-5-(3-chlorphenyl)-4-oxazolcarboxamid-hydrochlorid in 86,5%iger Ausbeute, Schmelzpunkt 229-231° (aus Methanol/Diäthyläther);
- aus t-Butyl-[2-[5-(4-chlorphenyl)-4-oxazolcarboxamido]äthyl]carbamat das N-(2-Aminoäthyl)-5-(4-chlorphenyl)-4-oxazolcarboxamid-hydrochlorid in 73,4%iger Ausbeute, Schmelzpunkt 274-275° (aus Methanol/Diäthyläther);
- aus t-Butyl-[2-[5-(2-fluorphenyl)-4-oxazolcarboxamido]äthyl]carbamat das N-(2-Aminoäthyl)-5-(2-fluorphenyl)-4-oxazolcarboxamid-hydrochlorid in 65,4%iger Ausbeute, Schmelzpunkt 271-272° (aus Methanol/Diäthyläther);
- aus t-Butyl-[2-[5-(3-fluorphenyl)-4-oxazolcarboxamido]äthyl]carbamat das N-(2-Aminoäthyl)-5-(3-fluorphenyl)-4-oxazolcarboxamid-hydrochlorid in 95%iger Ausbeute, Schmelzpunkt 258-260° (aus Methanol/Diäthyläther);

- aus t-Butyl-[2-[5-(4-fluorphenyl)-4-oxazolcarboxamido]äthyl]carbamat das N-(2-Aminoäthyl)-5-(4-fluorphenyl)-4-oxazolcarboxamid-hydrochlorid in 84%iger Ausbeute, Schmelzpunkt 287° (aus Methanol/Diäthyläther);
- aus t-Butyl-[2-[5-(4-bromphenyl)-4-oxazolcarboxamido]äthyl]carbamat das N-(2-Aminoäthyl)-5-(4-bromphenyl)-4-oxazolcarboxamid-hydrochlorid in 89%iger Ausbeute, Schmelzpunkt 254-256° (aus Methanol/Diäthyläther);
- aus t-Butyl-[2-[5-(3,4-dichlorphenyl)-4-oxazolcarboxamido]äthyl]carbamat das N-(2-Aminoäthyl)-5-(3,4-dichlorphenyl)-4-oxazolcarboxamid-hydrochlorid in 98%iger Ausbeute, Schmelzpunkt 275-276° (aus Methanol);
- aus t-Butyl-[2-[5-(2-furyl)-4-oxazolcarboxamido]äthyl]carbamat das N-(2-Aminoäthyl)-5-(2-furyl)-4-oxazolcarboxamid-hydrochlorid in 95%iger Ausbeute, Schmelzpunkt 238-239° (aus Methanol/Diäthyläther);
- aus t-Butyl-[2-[5-(2-thienyl)-4-oxazolcarboxamido]äthyl]carbamat das N-(2-Aminoäthyl)-5-(2-thienyl)-4-oxazolcarboxamid-hydrochlorid in 71%iger Ausbeute, Schmelzpunkt 263-264° (aus Methanol/Diäthyläther);
- aus t-Butyl-[2-[5-(5-brom-2-furyl)-4-oxazolcarboxamido]äthyl]carbamat das N-(2-Aminoäthyl)-5-(5-brom-2-furyl)-4-oxazolcarboxamid-hydrochlorid in 91%iger Ausbeute, Schmelzpunkt 231-233° (aus Methanol/Diäthyläther);
- aus t-Butyl-[2-[5-(2,4-difluorphenyl)-4-oxazolcarboxamido]äthyl]carbamat das N-(2-Aminoäthyl)-5-(2,4-difluorphenyl)-4-oxazolcarboxamid-hydrochlorid in 91%iger Ausbeute, Schmelzpunkt >290° (aus Methanol) und
- aus t-Butyl-[2-[5-(2,6-difluorphenyl)-4-oxazolcarboxamido]äthyl]carbamat das N-(2-Aminoäthyl)-5-(2,6-difluorphenyl)-4-oxazolcarboxamid-hydrochlorid in 80%iger Ausbeute, Schmelzpunkt 266-267° (aus Methanol).

Die als Ausgangsstoffe eingesetzten Carbamate wurden in analoger Weise wie in Beispiel 1 (E1) beschrieben hergestellt:

- aus 5-(2-Chlorphenyl)-4-oxazolcarbonsäureäthylester und t-Butyl-(2-aminoäthyl)carbamat das t-Butyl-[2-[5-(2-chlorphenyl)-4-oxazolcarboxamido]äthyl]carbamat in 38%iger Ausbeute (wurde ohne weitere Reinigung weiterverarbeitet);
- aus 5-(3-Chlorphenyl)-4-oxazolcarbonsäureäthylester und t-Butyl-(2-aminoäthyl)carbamat das t-Butyl-[2-[5-(3-chlorphenyl)-4-oxazolcarboxamido]äthyl]carbamat in 38%iger Ausbeute, Schmelzpunkt 140-141° (aus Essigester/Hexan);
- aus 5-(4-Chlorphenyl)-4-oxazolcarbonsäureäthylester und t-Butyl-(2-aminoäthyl)carbamat das t-Butyl-[2-[5-(4-chlorphenyl)-4-oxazolcarboxamido]äthyl]carbamat in 49%iger Ausbeute, Schmelzpunkt 164° (aus Essigester/Hexan);
- aus 5-(2-Fluorphenyl)-4-oxazolcarbonsäureäthylester und t-Butyl-(2-aminoäthyl)carbamat das t-Butyl-[2-[5-(2-fluorphenyl)-4-oxazolcarboxamido]äthyl]carbamat in 58%iger Ausbeute, Schmelzpunkt 130-133° (aus Essigester/Hexan);
- aus 5-(3-Fluorphenyl)-4-oxazolcarbonsäureäthylester und t-Butyl-(2-aminoäthyl)carbamat das t-Butyl-[2-[5-(3-fluorphenyl)-4-oxazolcarboxamido]äthyl]carbamat in 69%iger Ausbeute, Schmelzpunkt 135-136° (aus Essigester/Hexan);
- aus 5-(4-Fluorphenyl)-4-oxazolcarbonsäureäthylester und t-Butyl-(2-aminoäthyl)carbamat das t-Butyl-[2-[5-(4-fluorphenyl)-4-oxazolcarboxamido]äthyl]carbamat in 24%iger Ausbeute, Schmelzpunkt 146-148° (aus Essigester/Hexan);
- aus 5-(4-Bromphenyl)-4-oxazolcarbonsäureäthylester und t-Butyl-(2-aminoäthyl)carbamat das t-Butyl-[2-[5-(4-bromphenyl)-4-oxazolcarboxamido]äthyl]carbamat in 32%iger Ausbeute, Schmelzpunkt 170-171° (aus Essigester/Hexan);
- aus 5-(3,4-Dichlorphenyl)-4-oxazolcarbonsäureäthylester und t-Butyl-(2-aminoäthyl)carbamat das t-Butyl-[2-[5-3,4-dichlorphenyl)-4-oxazolcarboxamido]äthyl]carbamat in 72%iger Ausbeute, Schmelzpunkt 126-128° (aus Essigester/Hexan);
- aus 5-(2-Furyl)-4-oxazolcarbonsäureäthylester und t-Butyl-(2-aminoäthyl)carbamat das t-Butyl-[2-[5-(2-furyl)-4-oxazolcarboxamido]äthyl]carbamat in 32%iger Ausbeute, Schmelzpunkt 86-87° (aus Essigester/Hexan) und
- aus 5-(2-Thienyl)-4-oxazolcarbonsäureäthylester und t-Butyl-(2-aminoäthyl)carbamat das t-Butyl-[2-[5-(2-thienyl)-4-oxazolcarboxamido]äthyl]carbamat in 12%iger Ausbeute, Schmelzpunkt 109-110° (aus Essigester/Hexan).

Die folgenden Carbamate wurden in analoger Weise wie in Beispiel 1 (E) beschrieben hergestellt:

- aus 5-(5-Brom-2-furyl)-4-oxazolcarbonsäure und t-Butyl-(2-aminoäthyl)carbamat das t-Butyl-[2-[5-(5-brom-2-furyl)-4-oxazolcarboxamido]äthyl]carbamat in 97%iger Ausbeute, Schmelzpunkt 120-121° (aus Essigester/Hexan);
- aus 5-(2,4-Difluorphenyl)-4-oxazolcarbonsäure und t-Butyl-(2-aminoäthyl)carbamat das t-Butyl-[2-[5-(2,4-difluorphenyl)-4-oxazolcarboxamido]äthyl]carbamat in 92%iger Ausbeute, Schmelzpunkt 133° (aus

Essigester/Hexan) und
- aus 5-(2,6-Difluorphenyl)-4-oxazolcarbonsäure und t-Butyl-(2-aminoäthyl)carbamat das t-Butyl-[2-[5-(2,6-difluorphenyl)-4-oxazolcarboxamido]äthyl]carbamat in 61%iger Ausbeute, Schmelzpunkt 166-167° (aus Essigester/Hexan).

Die als Ausgangstoffe eingesetzten Carbonsäureäthylester wurden analog der in Tetrahedron Letters, 23, 235-236 (1982) beschriebenen Methode aus Isocyanessigsäureäthylester, den entsprechend substituierten Benzoesäuren bzw. Furancarbonsäure bzw.Thiophencarbonsäure, Phosphorsäurediphenylesterazid und Kaliumcarbonat hergestellt:
- aus 2-Chlorbenzoesäure und Isocyanessigsäureäthylester der 5-(2-Chlorphenyl)-4-oxazolcarbonsäureäthylester in 58%iger Ausbeute als gelbes Oel (wurde ohne weitere Reinigung weiterverarbeitet);
- aus 3-Chlorbenzoesäure und Isocyanessigsäureäthylester der 5-(3-Chlorphenyl)-4-oxazolcarbonsäureäthylester in 57%iger Ausbeute, Schmelzpunkt 57-58° (aus Essigester/Hexan);
- aus 4-Chlorbenzoesäure und Isocyanessigsäureäthylester der 5-(4-Chlorphenyl)-4-oxazolcarbonsäureäthylester in 76%iger Ausbeute, Schmelzpunkt 103-105° (aus Essigester/Hexan);
- aus 2-Fluorbenzoesäure und Isocyanessigsäureäthylester der 5-(2-Fluorphenyl)-4-oxazolcarbonsäureäthylester in 41%iger Ausbeute, Schmelzpunkt 62-63° (aus Essigester/Hexan);
- aus 3-Fluorbenzoesäure und Isocyanessigsäureäthylester der 5-(3-Fluorphenyl)-4-oxazolcarbonsäureäthylester in 84%iger Ausbeute, Schmelzpunkt 48-49° (aus Essigester/Hexan);
- aus 4-Fluorbenzoesäure und Isocyanessigsäureäthylester der 5-(4-Fluorphenyl)-4-oxazolcarbonsäureäthylester in 51%iger Ausbeute, Schmelzpunkt 53-54° (aus Essigester/Hexan);
- aus 4-Brombenzoesäure und Isocyanessigsäureäthylester der 5-(4-Bromphenyl)-4-oxazolcarbonsäureäthylester in 62%iger Ausbeute, Schmelzpunkt 118-119° (aus Essigester/Hexan);
- aus 3,4-Dichlorbenzoesäure und Isocyanessigsäureäthylester der 5-(3,4-Dichlorphenyl)-4-oxazolcarbonsäureäthylester in 42%iger Ausbeute, Schmelzpunkt 102-103° (aus Essigester/Hexan);
- aus 2-Furancarbonsäure und Isocyanessigsäureäthylester der 5-(2-Furyl)-4-oxazolcarbonsäureäthylester in 52%iger Ausbeute, Schmelzpunkt 97-98° (aus Essigester/Hexan);
- aus 2-Thiophencarbonsäure und Isocyanessigsäureäthylester der 5-(2-Thienyl)-4-oxazolcarbonsäureäthylester in 26%iger Ausbeute, Schmelzpunkt 41-42° (aus Essigester/Hexan);
- aus 5-Brom-2-furancarbonsäure und Isocyanessigsäureäthylester der 5-(5-Brom-2-furyl)-4-oxazolcarbonsäureäthylester in 32%iger Ausbeute, Schmelzpunkt 93° (aus Essigester/Hexan);
- aus 2,4-Difluorbenzoesäure und Isocyanessigsäureäthylester der 5-(2,4-Difluorphenyl)-4-oxazolcarbonsäureäthylester in 13%iger Ausbeute, Schmelzpunkt 76-77° (aus Essigester/Hexan) und
- aus 2,6-Difluorbenzoesäure und Isocyanessigsäureäthylester der 5-(2,6-Difluorphenyl)-4-oxazolcarbonsäureäthylester in 13%iger Ausbeute, als gelbes Oel, welches beim Stehenlassen kristallisiert.

Die folgenden Carbonsäuren wurden in analoger Weise wie in Beispiel 1 (D) beschrieben hergestellt:
- aus 5-(5-Brom-2-furyl)-4-oxazolcarbonsäureäthylester die 5-(5-Brom-2-furyl)carbonsäure in 66%iger Ausbeute, Schmelzpunkt 226-228° (aus Essigester/Hexan);
- aus 5-(2,4-Difluorphenyl)-4-oxazolcarbonsäureäthylester die 5-(2,4-Difluorphenyl)-4-oxazolcarbonsäure in 72%iger Ausbeute, Schmelzpunkt 180-182° (aus Wasser) und
- aus 5-(2,6-Difluorphenyl)-4-oxazolcarbonsäureäthylester die 5-(2,6-Difluorphenyl)-4-oxazolcarbonsäure in 64%iger Ausbeute (wurde ohne weitere Reinigung weiterverarbeitet).

## Beispiel 12

1,46 g (4,2 mMol) t-Butyl-[2-(2-amino-5-phenyl-4-oxazolcarboxamido)äthyl]carbamat werden in 50 ml Methylenchlorid gelöst und mit 5 ml Trifluoressigsäure versetzt. Das Reaktionsgemisch wird unter Rühren 2,5 Stunden zum Rückfluss erhitzt und danach unter vermindertem Druck eingedampft. Der Rückstand wird in Methanol gelöst, mit 5 ml 2M methanolischer Salzsäure versetzt und eingedampft. Umkristallisation des Rückstands aus Methanol/Diäthyläther liefert 1,3 g (94%) 2-Amino-N-(2-aminoäthyl)-5-phenyl-4-oxazolcarboxamiddihydrochlorid als weisse Kristalle, Schmelzpunkt 260°.

Das als Ausgangsmaterial eingesetzte t-Butyl-[2-(2-amino-5-phenyl-4-oxazolcarboxamido)äthyl]carbamat wurde wie folgt hergestellt:

Zu einer auf 0° abgekühlten Lösung von 16,1 g (152 mMol) Benzaldehyd und 20,0 g (140 mMol) Dichloressigsäuremethylester in 50 ml Aether tropft man innerhalb von 20 Minuten bei 0-5° eine Lösung von 3 g (0,13 Grammatome) Natrium in 50 ml Methanol zu. Danach wird das Reaktionsgemisch noch 1,5 Stunden bei 0-5° gerührt, dann mit 100 ml Essigester verdünnt und mit gesättigter Natriumchloridlösung

extrahiert. Die organische Phase wird über Magnesiumsulfat getrocknet und eingedampft. Der Rückstand wird in 50 ml Methanol gelöst und mit 6,72 g (112 mMol) Harnstoff 14 Stunden zum Rückfluss erhitzt. Das Reaktionsgemisch wird unter vermindertem Druck eingedampft, zwischen Methylenchlorid und Wasser verteilt, mit konz. Natronlauge auf pH 9 gestellt und extrahiert. Die alkalischen, wässrigen Phasen werden mit Methylenchlorid nachextrahiert. Die organischen Phasen werden vereinigt und 2 mal mit gesättigter Natriumchloridlösung gewaschen, getrocknet und eingedampft. Der Rückstand wird an 1 kg Kieselgel durch Mitteldruckchromatogaphie gereinigt. Eluieren mit Methylenchlorid, sowie Methylenchlorid/Methanol 9:1 liefert 8,4 g eines Oels, welches aus einem Gemisch von Methylenchlorid, Aether und Hexan kristallisiert. Man erhält auf diese Weise 4,7 g (35%) 2-Amino-5-phenyl-4-oxazolcarbonsäuremethylester als leicht gelbliche Kristalle, welche ohne weitere Reinigung weiterverarbeitet werden.

2,0 g (9,2 mMol) 2-Amino-5-phenyl-4-oxazolcarbonsäuremethylester werden mit 4,4 g (27,5 mMol) t-Butyl-(2-aminoäthyl)carbamat 6 Stunden bei 120° unter vermindetem Druck gerührt. Das Reaktionsgemisch wird dann an 200 g Kieselgel mit Essigester unter Zusatz von 5% Methanol als Eluierungsmittel chromatographiert. Die nach dem Dünnschichtchromatogramm reinen Fraktionen werden aus Methylenchlorid/Aether/Hexan kristallisiert und liefern 1,46 g (62%) t-Butyl-[2- (2-amino-5-phenyl-4-oxazolcarboxamido)äthyl]carbamat als weisse Kristalle, welche ohne weitere Reinigung verarbeitet werden.

## Beispiel 13

1,5 g (4,33 mMol) t-Butyl-[2-(5-amino-4-phenyl-3-isoxazolcarboxamido)äthyl]carbamat werden in 5 ml Methylenchlorid mit 1,7 ml Trifluoressigsäure 16 Stunden bei Raumtemperatur gerührt. Danach wird das Reaktionsgemisch unter vermindertem Druck eingedampft; der Rückstand wird in Aethanol gelöst, worauf man mit 2 ml äthanolischer Salzsäure (17,5% G/V) versetzt und wiederum eindampft. Umkristallisieren des Rückstands aus Methanol/Aether liefert 0,9 g (73,5%) 5-Amino-N-(2-aminoäthyl)-4-phenyl-3-isoxazolcarboxamid-hydrochlorid als weisse Kristalle, Schmelzpunkt 187-189°.

Das als Ausgangsmaterial eingesetzte t-Butyl-[2-(5-amino-4-phenyl-3-isoxazolcarboxamido)äthyl]-carbamat wurde wie folgt hergestellt:

Umsetzen von Benzylcyanid, Natriumäthylat und Oxalsäurediäthylester nach der in Org. Synth. Coll. Vol. II (1943) 287 und Chem. Ber. 107 (1974) 2794-2795 beschriebenen Methode in Alkohol, Ansäuern mit konz. (37%) Salzsäure auf pH 2 und anschliessendem Umkristallisieren des ausgefallenen Niederschlags aus Aethanol liefert Aethylphenylcyanpyruvat in 90%iger Ausbeute als gelbe Kristalle, Schmelzpunkt 127-128°.

5,0 g (23 mMol) Aethyl-phenylcyanpyruvat werden nach der in Chem. Ber. 107 (1974) 2794-2795 und Ber. Deutsch. Chem. Ges. 33 (1900) 2592-2595 beschriebenen Methode umgesetzt, wobei man 5-Amino-4-phenyl-3-isoxazolcarbonsäureäthylester in 69,7%iger Ausbeute als beige Kristalle erhält, welche nach Umkristallisation aus Essigester/Hexan bei 119-121° schmelzen.

2,9 g (12,5 mMol) 5-Amino-4-phenyl-3-isoxazolcarbonsäureäthylester werden mit 5,0 g (31,2 mMol) t-Butyl-(2-aminoäthyl)carbamat 2 Stunden unter vermindertem Druck auf 10° erwärmt, wobei das gebildete Aethanol laufend abdestilliert wird. Das erkaltete Reaktionsgemisch wird in Methylenchlorid gelöst und an 100 g Kieselgel chromatographiert. Eluieren mit Methylenchlorid, welches 5%, 10% bzw. 20% Essigester enthält, Zusammenfassen der reinen Fraktionen und Eindampfen liefert 1,9 g Kristalle. Umkristallisation aus Essigester/Hexan liefert 1,5 g (34,7%) t-Butyl-[2-(5-amino-4-phenyl-3-isoxazolcarboxamido)äthyl]carbamat als weisse Kristalle, Schmelzpunkt 144°.

## Beispiel 14

5,0 g (15,1 mMol) t-Butyl-[2-(4-phenyl-3-isoxazolcarboxamido)äthyl]carbamat werden analog Beispiel 13 mit Trifluoressigsäure 16 Stunden bei Raumtemperatur gerührt. Nach dem Einengen erhält man einen Rückstand, welcher in das Hydrochlorid übergeführt wird. Umkristallisation des Rohprodukts aus Methanol/Aether liefert 3,8 g (94,1%) N-(2-Aminoäthyl)-4-phenyl-3-isoxazolcarboxamid-hydrochlorid als weisse Kristalle, Schmelzpunkt 211-212°.

Das als Ausgangsmaterial eingesetzte t-Butyl-[2-(4-phenyl-3-isoxazolcarboxamido)äthyl]carbamat wurde wie folgt hergestellt:

7,6 g (32,72 mMol) 5-Amino-4-phenyl-3-isoxazolcarbonsäureäthylester werden in analoger Weise wie in

J. Org. Chem. 50 (13) 1985, 2372-2375 beschrieben in 160 ml Eisessig, 50 ml Wasser und 80 ml Tetrahydrofuran, bei 15-20° unter Rühren innerhalb 1 Stunde portionenweise mit insgesamt 22,6 g Natriumnitrit versetzt. Das Reaktionsgemisch wird danach auf 1 Liter Wasser gegossen und 3 mal mit je 400 ml Methylenchlorid extrahiert. Die organsichen Phasen werden vereinigt und zunächst zweimal mit je 1l gesättigter Natriumbicarbonatlösung und dann einmal mit 1l Wasser gewaschen, getrocknet, filtriert und im Vakuum eingeengt, wobei man nach Chromatographie an Kieselgel und Eluieren mit Methylenchlorid 3,9 g (55%) 4-Phenyl-3-isoxazolcarbonsäureäthylester als gelbes Oel erhält, welches ohne weitere Reinigung verwendet wird.

3,9 g (17,95 mMol) 4-Phenyl-3-isoxazolcarbonsäureäthylester und 5,8 g (36,2 mMol) t-Butyl-(2-aminoäthyl)carbamat werden zusammen 2 Stunden unter vermindertem Druck auf 110° erwärmt. Chromatographie an Kieselgel und Eluieren mit Methylenchlorid, welches 5% bzw. 10% Essigester enthält, liefert ein Oel, welches aus Essigester/Hexan kristallisiert. Man erhält auf diese Weise 5,0 g (84%) t-Butyl-[2-4-phenyl-3-isoxazolcarboxamido)äthyl]carbamat als weisse Kristalle, Schmelzpunkt 170-171°.

## Beispiel 15

In analoger Weise wie in den Beispielen 13 und 14 beschrieben wurden die folgenden Verbindungen hergestellt:
- aus t-Butyl-[2-[4-(2-chlorphenyl)-3-isoxazolcarboxamido]äthyl]carbamat das N-(2-Aminoäthyl)-4-(2-chlorphenyl)-3-isoxazolcarboxamid-hydrochlorid in 32,8%iger Ausbeute, Schmelzpunkt 255-256° (aus Methanol/Diäthyläther);
- aus t-Butyl-[2-[4-(3-chlorphenyl)-3-isoxazolcarboxamido]äthyl]carbamat das N-(2-Aminoäthyl)-4-(3-chlorphenyl)--3-isoxazolcarboxamid-hydrochlorid in 93%iger Ausbeute, Schmelzpunkt 181-183° (aus Methanol/Diäthyläther);
- aus t-Butyl-[2-[4-(4-chlorphenyl)-3-isoxazolcarboxamido]äthyl]carbamat das N-(2-Aminoäthyl)-4-(4-chlorphenyl)-3-isoxazolcarboxamid-hydrochlorid in 72%iger Ausbeute, Schmelzpunkt 254-256° (aus Methanol/Diäthyläther);
- aus t-Butyl-[2-[4-(3-fluorphenyl)-3-isoxazolcarboxamido]äthyl]carbamat das N-(2-Aminoäthyl)-4-(3-fluorphenyl)-3-isoxazolcarboxamid-hydrochlorid in 91,7%iger Ausbeute, Schmelzpunkt 183-186° (aus Methanol/Diäthyläther) und
- aus t-Butyl-[2-[4-(4-fluorphenyl)-3-isoxazolcarboxamido]äthyl]carbamat das N-(2-Aminoäthyl)-4-(4-fluorphenyl)-3-isoxazolcarboxamid-hydrochlorid (5:6). 0,3 Mol $H_2O$ in 30%iger Ausbeute, Schmelzpunkt 237-239° (aus Aethanol).

Die als Ausgangstoffe eingesetzten Carbamate wurden wie folgt hergestellt:
- aus 4-(2-Chlorphenyl)-3-isoxazolcarbonsäureäthylester und t-Butyl-2-(aminoäthyl)carbamat das t-Butyl-[2-[4-(2-chlorphenyl)-3-isoxazolcarboxamido]äthyl]carbamat in 87%iger Ausbeute, Schmelzpunkt 132-133° (aus Essigester/Hexan);
- aus 4-(3-Chlorphenyl)-3-isoxazolcarbonsäureäthylester und t-Butyl-(2-aminoäthyl)carbamat das t-Butyl-[2-[4-(3-chlorphenyl)-3-isoxazolcarboxamido]äthyl]carbamat in 78,6%iger Ausbeute, Schmelzpunkt 147-148° (aus Essigester/Hexan);
- aus 4-(4-Chlorphenyl)-3-isoxazolcarbonsäureäthylester und t-Butyl-(2-aminoäthyl)carbamat das t-Butyl-[2-[4-(4-chlorphenyl)-3-isoxazolcarboxamido]äthyl]carbamat in 87%iger Ausbeute, Schmelzpunkt 135-137° (aus Essigester/Hexan);
- aus 4-(3-Fluorphenyl)-3-isoxazolcarbonsäureäthylester und t-Butyl-(2-aminoäthyl)carbamat das t-Butyl-[2-[4-(3-fluorphenyl)-3-isoxazolcarboxamido]äthyl]carbamat in 75%iger Ausbeute, Schmelzpunkt 162-163° (aus Aethanol/Diäthyläther) und
- aus 4-(4-Fluorphenyl)-3-isoxazolcarbonsäureäthylester und t-Butyl-(2-aminoäthyl)carbamat das t-Butyl-[2-[4-(4-fluorphenyl)-3-isoxazolcarboxamido]äthyl]carbamat in 95,9%iger Ausbeute, Schmelzpunkt 140-141° (aus Essigester/Hexan) .

Die als Ausgangsstoffe eingesetzten substituierten 3-Isoxazolcarbonsäureäthylester wurden aus den entsprechenden substituierten 5-Amino-3-isoxazolcarbonsäureestern hergestellt, welche ihrerseits aus den entsprechenden ß-Cyan-α-oxo-dihydrozimtsäureestern erhalten wurden:
Der 4-(2-Chlorphenyl)-3-isoxazolcarbonsäureäthylester in 52%iger Ausbeute als rotes Oel;
der 4-(3-Chlorphenyl)-3-isoxazolcarbonsäureäthylester in 73%iger Ausbeute als rotes Oel;
der 4-(4-Chlorphenyl)-3-isoxazolcarbonsäureäthylester in 51%iger Ausbeute als oranges Oel;
der 4-(3-Fluorphenyl)-3-isoxazolcarbonsäureäthylester in 65%iger Ausbeute als gelbes Oel;

der 4-(4-Fluorphenyl)-3-isoxazolcarbonsäureäthylester in 42%iger Ausbeute, Schmelzpunkt 57-58° (aus Essigester/Hexan);

der 5-Amino-4-(2-chlorphenyl)-3-isoxazolcarbonsäureäthylester in 76%iger Ausbeute, Schmelzpunkt 141-142° (aus Essigester/Hexan);

der 5-Amino-4-(3-chlorphenyl)-3-isoxazolcarbonsäureäthylester in 22%iger Ausbeute, Schmelzpunkt 151-152° (aus Essigester/Hexan);

der 5-Amino-4-(4-chlorphenyl)-3-isoxazolcarbonsäureäthylester in 52%iger Ausbeute, Schmelzpunkt 132-133° (aus Essigester/Hexan);

der 5-Amino-4-(3-fluorphenyl)-3-isoxazolcarbonsäureäthylester in 75%iger Ausbeute, Schmelzpunkt 119° (aus Essigester/Hexan) und

der 5-Amino-4-(4-fluorphenyl)-3-isoxazolcarbonsäureäthylester in 75%iger Ausbeute, Schmelzpunkt 137-138° (aus Essigester/Hexan).

Die als Ausgangsstoffe eingesetzten Dihydrozimtsäureester wurden nach der in Org. Synth. Coll. Vol. II (1943) 287 und Chem. Ber. 107 (1974) 2794-2795 beschriebenen Methode aus den entsprechenden Benzylcyaniden, Natriumäthylat und Oxalsäurediäthylester hergestellt:

Der 2-Chlor-ß-cyan-α-oxo-dihydrozimtsäureäthylester in 67%iger Ausbeute, Schmelzpunkt 133-135° (aus Aethanol/Wasser);

der 3-Chlor-ß-cyan-α-oxo-dihydrozimtsäureäthylester in 90%iger Ausbeute, Schmelzpunkt 76-77° (aus Aethanol/Wasser);

der 4-Chlor-ß-cyan-α-oxo-dihydrozimtsäureäthylester in 89,5%iger Ausbeute, Schmelzpunkt 137-139° (aus Wasser);

der 2-Fluor-ß-cyan-α-oxo-dihydrozimtsäureäthylester in 85%iger Ausbeute, Schmelzpunkt 94-95° (aus Aethanol/Wasser) und

der 4-Fluor-ß-cyan-α-oxo-dihydrozimtsäureäthylester in 90%iger Ausbeute, Schmelzpunkt 142-144° (aus Aethanol/Wasser).

## Beispiel 16

5,0 g (15,1 mMol) t-Butyl-[2-(4-phenyl-3-pyrazolcarboxamido)äthyl]carbamat werden mit 6 ml (78,4 mMol) Trifluoressigsäure und 10 ml Methylenchlorid 16 Stunden bei Raumtemperatur gerührt und danach unter vermindertem Druck eingedampft. Der Rückstand wird mit 7 ml äthanolischer Salzsäure (17,5% G/V) versetzt und eingedampft. Umkristallisation des Rückstands aus Methanol liefert 3,1 g (76,8%) N-(2-Aminoäthyl)-4-phenyl-3-pyrazolcarboxamid-hydrochlorid als weisse Kristalle, Schmelzpunkt 285-286°.

Das als Ausgangsmaterial eingesetzte t-Butyl-[2-(4-phenyl-3-pyrazolcarboxamido)äthyl]carbamat wurde wie folgt hergestellt:

(A) Benzaldehyd, N, N-Dimethylglycinäthylester und Natriumhydrid werden in Aether unter Zusatz einer katalytischen Menge von Aethanol nach der in Lieb. Ann. Chem. 703 (1967) 37-43 beschriebenen Methode zu α-Dimethylaminozimtsäureäthylester umgesetzt. Dieser wird dann nach der in Tetrahedron Letters, 46, (1978) 4573-4574 beschriebenen Methode mit Oxalylchlorid und Hydrazin in 60%iger Ausbeute zum 4-Phenyl3-pyrazolcarbonsäureäthylester umgesetzt, Schmelzpunkt 164-165° nach Umkristallisation aus Aethanol/Diäthyläther.

In analoger Weise wie oben beschrieben wurden die folgenden 3-Pyrazolcarbonsäureester hergestellt; zur Herstellung der 1-Methylsubstituierten Verbindungen wurde N-Methylhydrazin anstelle von Hydrazin verwendet, wobei jeweils die entstandenen Gemische der 1- und 2-Methylderivate durch Chromatographie and Kieselgel mit Methylenchlorid getrennt wurden:

Der 4-(3-Fluorphenyl)-3-pyrazolcarbonsäureäthylester in 46%iger Ausbeute, Schmelzpunkt 161-162° (aus Aethanol/Diäthyläther);

der 4-(4-Fluorphenyl)-3-pyrazolcarbonsäureäthylester in 33,7%iger Ausbeute, Schmelzpunkt 177-179° (aus Aethanol/Diäthyläther);

der 1-Methyl-4-phenyl-3-pyrazolcarbonsäureäthylester in 27%iger Ausbeute, Schmelzpunkt 92° (aus Essigester/Hexan);

der 4-(3-Fluorphenyl)-1-methyl-3-pyrazolcarbonsäureäthylester in 20%iger Ausbeute, Schmelzpunkt 93-94° (aus Essigester/Hexan) und

der 4-(4-Fluorphenyl)-1-methyl-3-pyrazolcarbonsäureäthylester in 28%iger Ausbeute, Schmelzpunkt 58-59° (aus Diäthyläther/Petroläther).

(B) 5,0 g (23,12 mMol) 4-Phenyl-3-pyrazolcarbonsäureäthylester werden mit 7,4 g (46,19 mMol) t-Butyl-(2-aminoäthyl)carbamat unter vermindertem Druck 2 Stunden auf 120 erwärmt, wobei das gebildete Aethanol laufend abdestilliert wird. Dann wird das Reaktionsgemisch in Methylenchlorid aufgenommen und mit einem 9:1-, 8:2- und 7:3-Gemisch von Methylenchlorid und Essigester als Eluierungsmittel an 150 g Kieselgel chromatographiert. Die nach dem Dünnschichtchromatogramm reinen Fraktionen werden vereinigt und eingedampft, und der Rückstand aus Essigester/Hexan kristallisiert, wobei 5,1 g (66,8%) t-Butyl-[2-(4-phenylpyrazol-3-carboxamido)äthyl]carbamat als weisse Kristalle erhalten werden, Schmelzpunkt 71°/162-163° (enth. noch 0,05 Mol Hexan).

## Beispiel 17

4,4 g (12,63 mMol) t-Butyl-[2-(4-(3-fluorphenyl)-3-pyrazolcarboxamido)äthyl]carbamat werden in analoger Weise wie in Beispiel 16 mit Trifluoressigsäure 16 Stunden bei Raumtemperatur gerührt. Danach wird das Reaktionsgemisch eingedampft, und der Rückstand in das Hydrochlorid übergeführt, welches aus Methanol/Diäthyläther umkristallisiert wird. Nach dem Trocknen erhält man 2,7 g (75%) N-(2-Aminoäthyl)-4-(3-fluorphenyl)-3-pyrazolcarboxamid-hydrochlorid als weisse Kristalle, Schmelzpunkt 284-285°.

Das als Ausgangsmaterial eingesetzte t-Butyl-[2-(4-(3-fluorphenyl)-3-pyrazolcarboxamido)äthyl]-carbamat, wurde wie folgt hergestellt:

In analoger Weise wie in Beispiel 16 beschrieben wurden 4,6 g (19,64 mMol) 4-(3-Fluorphenyl)-3-pyrazolcarbonsäureäthylester mit t-Butyl-(2-aminoäthyl)carbamat umgesetzt. Chromatographieren des Rohprodukts und Umkristallisieren aus Essigester/Hexan liefert 4,4 g (64,6%) t-Butyl-[2-(4-(3-fluorphenyl)-3-pyrazolcarboxamido)äthyl]carbamat als weisse Kristalle, Schmelzpunkt 175-176°.

## Beispiel 18

In analoger Weise wie in den Beispielen 16 und 17 beschrieben erhält man aus 6,4 g (18,37 mMol) t-Butyl-[2-(4-(4-fluorphenyl)-3-pyrazolcarboxamido)äthyl]carbamat nach Abspaltung der t-Butoxycarbonylgruppe mit Trifluoressigsäure und Aufarbeiten 6,2 g des erwünschten Hydrochlorids als Rohprodukt. Umkristallisation aus Methanol/Aether liefert 4,2 g (80%) N-(2-Aminoäthyl)-4-(4-fluorphenyl)-3-pyrazolcarboxamid-hydrochlorid als weisse Kristalle, Schmelzpunkt 271-273°.

Das als Ausgangsmaterial eingesetzte t-Butyl-[2-(4-(4-fluorphenyl)-3-pyrazolcarboxamido)äthyl]carbamat wurde wie folgt hergestellt:

In analoger Weise wie in den Beispielen 16 und 17 beschrieben wurden aus 6,1 g (26 mMol) 4-(4-Fluorphenyl)-3-pyrazolcarbonsäureäthylester 6,4 g (67,2%) t-Butyl-[2-(4-(4-fluorphenyl)-3-pyrazolcarboxamido)äthyl]carbamat als weisse Kristalle erhalten, welche nach Umkristallisation aus Essigester/Hexan bei 162-163° schmelzen.

## Beispiel 19

In analoger Weise wie in den Beispielen 16 und 17 beschrieben erhält man aus 4,6 g (13,4 mMol) t-Butyl-[2-(1-methyl-4-phenyl-3-pyrazolcarboxamido)äthyl]carbamat nach Abspaltung der t-Butoxycarbonylgruppe mit Trifluoressigsäure und Aufarbeitung 4,8 g des rohen Hydrochlorids. Umkristallisation aus Aethanol/Diäthyläther liefert 3,4 g (90%) N-(2-Aminoäthyl)-1-methyl-4-phenyl-3-pyrazolcarboxamid-hydrochlorid als weisse Kristalle, Schmelzpunkt 182°.

Das als Ausgangsmaterial eingesetzte t-Butyl-[2-(1-methyl-4-phenyl-3-pyrazolcarboxamido)äthyl]-carbamat wurde wie folgt hergestellt:

In analoger Weise wie in den Beispielen 16 und 17 beschrieben erhält man aus 6,6 g (28,7 mMol) 1-Methyl-4-phenyl-3-pyrazolcarbonsäureäthylester 4,6 g (71,5%) t-Butyl-[2-(1-methyl-4-phenyl-3-pyrazolcarboxamido)äthyl]carbamat als weisse Kristalle, Schmelzpunkt 145° (aus Essigester/Hexan).

## Beispiel 20

3,4 g (9,38 mMol) t-Butyl-[2-[4-(3-fluorphenyl)-1-methyl-3-pyrazolcarboxamido]äthyl]carbamat werden in analoger Weise wie in Beispiel 16 beschrieben 16 Stunden mit Trifluoressigsäure bei Raumtemperatur gerührt. Das Reaktionsgemisch wird danach eingedampft, und der Rückstand in das Hydrochlorid übergeführt. Umkristallisation aus Aethanol/Diäthyläther liefert 2,6 g (92,8%) N-(2-Aminoäthyl)-4-(3-fluorphenyl)-1-methyl-3-pyrazolcarboxamid-hydrochlorid als weisse Kristalle, Schmelzpunkt 160-161°.

Das als Ausgangsmaterial eingesetzte t-Butyl-[2-(4-(3-fluorphenyl)-1-methyl-3-pyrazolcarboxamido)-äthyl]carbamat wurde wie folgt hergestellt:

3,4 g (13,7 mMol) 4-(3-Fluorphenyl)-1-methyl-3-pyrazolcarbonsäureäthylester und 5,5 g (34,32 mMol) t-Butyl-(2-aminoäthyl)carbamat werden unter vermindertem Druck 18 Stunden bei 130° gerührt und anschliessend an 100 g Kieselgel chromatographiert. Eluieren mit einem 9:1-, 8:2- und 7:3-Gemisch von Methylenchlorid und Essigester liefert 3,9 g Kristalle, welche aus Essigester/Hexan umkristallisiert werden. Auf diese Weise erhält man 3,4 g (68,5%) t-Butyl-[2-[4-(3-fluorphenyl)-1-methyl-3-pyrazolcarboxamido]-äthyl]carbamat als weisse Kristalle, Schmelzpunkt 124°.

## Beispiel 21

9,8 g (27,0 mMol) t-Butyl-[2-[4-(4-fluorphenyl)-1-methyl-3-pyrazolcarboxamido]äthyl]carbamat werden in analoger Weise wie in Beispiel 16 beschrieben 16 Stunden bei Raumtemperatur mit Trifluoressigsäure gerührt. Danach wird das Reaktionsgemisch eingedampft und der Rückstand in das Hydrochlorid übergeführt. Umkristallisation aus Aethanol/Diäthyläther liefert 7,5 g (92,8%) N-(2-Aminoäthyl)-4-(4-fluorphenyl)-1-methyl-3-pyrazolcarboxamid-hydrochlorid als weisse Kristalle, Schmelzpunkt 195-196°.

Das als Ausgangsmaterial eingesetzte t-Butyl-[2-[4-(4-fluorphenyl)-1-methyl-3-pyrazolcarboxamido]-äthyl]carbamat wurde wie folgt hergestellt:

8,6 g (34,6 mMol) 4-(4-Fluorphenyl)-1-methyl-3-pyrazolcarbonsäureäthylester werden während 60 Minuten mit 90 ml 2N Natronlauge und 50 ml Wasser auf 70° erwärmt. Ueberführung in die Säureform und Umkristallisation des Rohprodukts aus Essigester/Hexan liefert 6,9 g (90,4%) 4-(4-Fluorphenyl)-1-methyl-3-pyrazolcarbonsäure als weisse Kristalle, Schmelzpunkt 146-147°.

6,9 g (31,34 mMol) 4-(4-Fluorphenyl)-1-methyl-3-pyrazolcarbonsäure werden mit 400 ml Tetrahydrofuran und 5,2 g (32 mMol) 1,1′-Carbonyldiimidazol 2 Stunden zum Rückfluss erhitzt. Danach werden 5,3 g (33 mMol) t-Butyl-(2-aminoäthyl)carbamat zugegeben. Das Reaktionsgemisch wird weitere 2 Stunden zum Rückfluss erhitzt und danach eingedampft. Der Rückstand wird zwischen Essigester und Wasser verteilt, die organische Phase mehrmals mit Wasser gewaschen, über Magnesiumsulfat getrocknet und eingedampft. Umkristallisation des kristallienen Rückstands aus Essigester/Hexan liefert 9,8 g (86,3%) t-Butyl-[2-[4-(4-fluorphenyl)-1-methyl-3-pyrazolcarboxamido]äthyl]carbamat als weisse Kristalle, Schmelzpunkt 144°.

## Beispiel 22

Aus 7,2 g (18,78 mMol) t-Butyl-[2-[5-(3,5-difluorphenyl)-4-thiazolcarboxamido]äthyl]carbamat wurden in analoger Weise wie in Beispiel 1 beschrieben 5,7 g (95%) N-(2-Aminoäthyl)-5-(3,5-difluorphenyl)-4-thiazolcarboxamid-hydrochlorid als weisse Kristalle erhalten, Schmelzpunkt 266-267° (aus Methanol).

Das als Ausgangsmaterial eingesetzte t-Butyl-[2-[5-(3,5-difluorphenyl)-4-thiazolcarboxamido]äthyl]-carbamat wurde wie folgt hergestellt:

25,0 g (179,7 mMol) 3,5-Difluorbenzonitril werden in 250 ml Aethanol gelöst und auf -10° abgekühlt. Danach leitet man während 20 Minuten Salzsäuregas ein, wobei die Innentemperatur auf 30° ansteigt. Man rührt danach 1 Stunde bei Raumtemperatur und leitet zwecks Entfernung überschüssiger Salzsäure während 30 Minuten Stickstoff durch das Reaktionsgemisch. Man kühlt auf 0° ab und fügt 200 ml Aether zu. Die trübe Lösung lässt man 5 Tage bei ca. 0° stehen und filtriert danach die Kristalle ab. Man erhält nach Trocknen bei 50° im Vakuum 27,8 g (70%) 3,5-Difluorbenziminoäthyläther-hydrochlorid als weisse Kristalle vom Schmelzpunkt 133-134°.

300 ml Pyridin werden auf 0-5° abgekühlt. Man leitet 5-10 g Schwefelwasserstoff ein, fügt dann 27,5 g (124 mMol) 3,5-Difluorbenziminoäthyläther-hydrochlorid zu und lässt das Reaktionsgemisch verschlossen während ca. 48 Stunden bei Raumtemperatur stehen. Man entfernt den überschüssigen Schwefelwasserstoff durch Austreiben mit Stickstoff und engt dann das Reaktionsgemisch im Vakuum ein. Der Rückstand wird unter Rühren mit 500 ml Hexan versetzt, worauf man auf 5° abkühlt. Nach 30 Minuten wird von den

unlöslichen Teilen abgenutscht. Die gelbe, klare Lösung wird im Vakuum eingeengt. Man erhält als Rückstand 23,8 g (95%) 3,5-Difluorthiobenzoesäure-o-äthylester, als oranges übelriechendes Oel.

Wie in Beispiel 1 (C) beschrieben, erhält man aus 3,5-Difluorthiobenzoesäure-o-äthylester in 82%iger Ausbeute 5-(3,5-Difluorphenyl)-4-thiazolcarbonsäureäthylester, welcher nach Umkristallisation aus Essigester/Hexan bei 112-113° schmilzt.

Der 5-(3,5-Difluorphenyl)-4-thiazolcarbonsäureäthylester wird in analoger Weise wie in Beispiel 1 (D) beschrieben verseift, wobei man nach Ansäuren die 5-(3,5-Difluorphenyl)-4-thiazolcarbonsäure in g7%iger Ausbeute erhält, Schmelzpunkt 200° (aus Wasser).

Aus 5-(3,5-Difluorphenyl)-4-thiazolcarbonsäure erhält man in analoger Weise wie in Beispiel 1 (E) beschrieben t-Butyl-[2-[5-(3,5-difluorphenyl)-4-thiazolcarboxamido]äthyl]carbamat in 88%iger Ausbeute als weisse Kristalle vom Schmelzpunkt 141-142°.

## Beispiel 23

(A) 6,3 g (13,1 mMol) t-Butyl-[2-[5-(3-jodphenyl)-4-thiazolcarboxamido]äthyl]carbamat werden in 70 ml Methylenchlorid gelöst, mit 5,1 ml Trifluoressigsäure versetzt und 2 Stunden unter Rückfluss gerührt. Anschliessend wird unter vermindertem Druck eingeengt. Der Rückstand wird in 50 ml Methanol gelöst und mit 5,2 ml 2,7N äthanolischer Salzsäure versetzt, wobei man 4,8 g (88%) N-(2-Aminoäthyl)-5-(3-jodphenyl)-4-thiazolcarboxamid-hydrochlorid als weisse Kristalle erhält, Schmelzpunkt 272-273°.

Das als Ausgangsmaterial eingesetzte t-Butyl-[2-[5-(3-jodphenyl)-4-thiazolcarboxamido]äthyl]carbamat wurde wie folgt hergestellt:

(B) 52,5 g (0,2 Mol) 3-Jodbenzoesäuremethylester werden mit 81 g (0,2 Mol) Lawesson-Reagens in 330 ml Xylol 18 Stunden zum Rückfluss erhitzt. Durch Aufarbeiten in analoger Weise wie in Beispiel 1(B) beschrieben, erhält man 47 g (84,4%) 3-Jodthiobenzoesäure-O-methylester als gelbes übelriechendes Oel, welches ohne weitere Reinigung verwendet wird.

(C) Analog der in Synthesis 10, (1976) 681-682 beschriebenen Methode werden 47 g (169 mMol) 3-Jodthiobenzoesäure-O-methylester in Gegenwart von 1-5% pulverisiertem Kaliumhydroxid in Aethanol mit Isocyanessigsäureäthylester umgesetzt. Umkristallisation aus Aether liefert 45,8 g (75,4%) 5-(3-Jodphenyl)-4-thiazolcarbonsäureäthylester als ockergelbe Kristalle, Schmelzpunkt 77-78°.

(D) 20 g (55,68 mMol) 5-(3-Jodphenyl)-4-thiazolcarbonsäureäthylester werden in analoger Weise wie in Beispiel 1(D) beschrieben hydrolysiert, wobei man 17,6 g (95,5%) 5-(3-Jodphenyl)-4-thiazolcarbonsäure als gelbe Kristalle erhält, Schmelzpunkt 186°.

(E) Umsetzen von 5,0 g (15,1 mMol) 5-(3-Jodphenyl)-4-thiazolcarbonsäure mit 2,6 g (16,2 mMol) t-Butyl-(2-aminoäthyl)carbamat in analoger Weise wie in Beispiel 1(E) beschrieben liefert 6,3 g (88,1%) t-Butyl-[2-[5-(3-jodphenyl)-4-thiazolcarboxamido]äthyl]carbamat als weisse Kristalle, Schmelzpunkt 111-112°.

## Beispiel A

In an sich bekannter Weise können nach jedem Fachmann geläufigen Methoden Lacktabletten folgender Zusammensetzung hergestellt werden:

| Kern | |
|---|---|
| N-(2-Aminoäthyl)-5-phenyl-4-thiazolcarboxamid-hydrochlorid | 100,00 mg |
| Milchzucker pulv. | 148,00 mg |
| Maisstärke | 120,00 mg |
| Polyvinylpyrrolidon | 20,00 mg |
| Natrium-carboxymethylstärke | 10,00 mg |
| Magnesiumstearat | 2,00 mg |
| Kerngewicht | 400,00 mg |
| Lackschicht | |
| Hydroxypropylmethylcellulose | 5,00 mg |
| Talk | 3,76 mg |
| Titandioxid | 1,00 mg |
| Eisenoxid gelb | 0,20 mg |
| Eisenoxid siena | 0,04 mg |
| Gesamtgewicht einer Lacktablette | 410,00 mg |

## Beispiel B

In an sich bekannter Weise können nach jedem Fachmann geläufigen Methoden Lacktabletten folgender Zusammensetzung hergestellt werden:

| | |
|---|---|
| N-(2-Aminoäthyl)-5-phenyl-4-thiazolcarboxamid-hydrochlorid | 150,00 mg |
| Milchzucker pulv. | 148,00 mg |
| Maisstärke | 60,00 mg |
| Polyvinylpyrrolidon | 25,00 mg |
| Natrium-carboxymethylstärke | 15,00 mg |
| Magnesiumstearat | 2,50 mg |
| Kerngewicht | 400,00 mg |
| Lackschicht | |
| Hydroxypropylmethylcellulose | 4,50 mg |
| Aethylcellulose | 1,50 mg |
| Polyäthylenglycol 6000 | 0,60 mg |
| Talk | 2,40 mg |
| Titandioxid | 2,90 mg |
| Eisenoxid gelb | 0,10 mg |
| Gesamtgewicht einer Lacktablette | 412,00 mg |

## Beispiel C

In Analogie zu Beispielen A und B können aus den folgenden, ebenfalls bevorzugten Verbindungen Lacktabletten hergestellt werden:

N-(2-Aminoäthyl)-5-(2-fluorphenyl)-4-thiazolcarboxamidhydrochlorid;
N-(2-Aminoäthyl)-5-(3-fluorphenyl)-4-thiazolcarboxamidhydrochlorid;
N-(2-Aminoäthyl)-5-(4-fluorphenyl)-4-thiazolcarboxamidhydrochlorid;
N-(2-Aminoäthyl)-5-(4-chlorphenyl)-4-oxazolcarboxamidhydrochlorid;

N-(2-Aminoäthyl)-5-(2-furyl)-4-oxazolcarboxamidhydrochlorid;
N-(2-Aminoäthyl)-5-(3,5-dichlorphenyl)-4-thiazolcarboxamid-hydrochlorid;
N-(2-Aminoäthyl)-5-(2,4-difluorphenyl)-4-thiazolcarboxamid-hydrochlorid und
N-(2-Aminoäthyl)-5-(3,5-difluorphenyl)-4-thiazolcarboxamid-hydrochlorid.

## Ansprüche

1. Aethylendiaminmonoamidderivate der allgemeinen Formel
$R\text{-}CO\text{-}NH\text{-}CH_2\text{-}CH_2\text{-}NH_2$ I
worin R eine der Gruppen

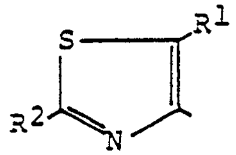

(a)

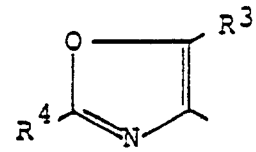

(b)

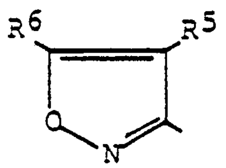

(c)

und

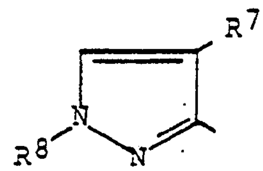

(d)

bedeutet, worin $R^1$ gegebenenfalls durch Halogen, nieder- Alkyl, nieder-Alkoxy, Trifluormethyl, Cyan oder Aryl-nieder-alkoxy monosubstituiertes Phenyl, Dihalophenyl, Furyl oder gegebenenfalls durch Halogen monosubstituiertes Thienyl, $R^2$ Wasserstoff, Halogen oder Amino, $R^3$, $R^5$ und $R^7$ je gegebenenfalls durch Halogen mono-oder di-substituiertes Phenyl, Thienyl oder gegebenenfalls durch Halogen monosubstituiertes Furyl, $R^4$ und $R^6$ je Wasserstoff oder Amino und $R^8$ Wasserstoff oder nieder-Alkyl bedeuten,
sowie pharmazeutisch verwendbare Säureadditionssalze davon.

2. Verbindungen gemäss Anspruch 1, worin R die Gruppe (a) oder (b) bedeutet.

3. Verbindungen gemäss Anspruch 2, worin $R^1$ gegebenenfalls durch Halogen, nieder-Alkyl, nieder-Alkoxy, Trifluormethyl oder Cyan monosubstituiertes Phenyl, Dihalophenyl, Furyl oder gegebenenfalls durch Halogen monosubstituiertes Thienyl und $R^2$ Wasserstoff oder Amino bedeuten bzw. $R^3$ durch Halogen mono- oder di-substituiertes Phenyl, Thienyl oder gegebenenfalls durch Halogen monosubstituiertes Furyl und $R^4$ Wasserstoff bedeuten.

4. Verbindungen gemäss Anspruch 3, worin $R^1$ durch Halogen monosubstituiertes Phenyl oder Dihalophenyl und $R^2$ Wasserstoff bedeuten bzw. $R^3$ durch Halogen mono- oder di-substituiertes Phenyl und $R^4$ Wasserstoff bedeuten.

5. N-(2-Aminoäthyl)-5-phenyl-4-thiazolcarboxamid.

6. N-(2-Aminoäthyl)-5-(3-fluorphenyl)-4-thiazolcarboxamid.

7. N-(2-Aminoäthyl)-5-(3,5-difluorphenyl)-4-thiazolcarboxamid.

8. N-(2-Aminoäthyl)-5-(2-fluorphenyl)-4-thiazolcarboxamid, N-(2-Aminoäthyl)-5-(4-fluorphenyl)-4-thiazolcarboxamid, N-(2-Aminoäthyl)-5-(4-chlorphenyl)-4-oxazolcarboxamid, N-(2-Aminoäthyl)-5-(2-furyl)-4-oxazolcarboxamid, N-(2-Aminoäthyl)-5-(3,5-dichlorphenyl)-4-thiazolcarboxamid, N-(2-Aminoäthyl)-5-(2,4-difluorphenyl)-4-thiazolcarboxamid, N-(2-Aminoäthyl)-5-(4-fluorphenyl)-4-oxazolcarboxamid, N-(2-Aminoäthyl)-5-(4-bromphenyl)-4-oxazolcarboxamid, N-(2-Aminoäthyl)-4-(3-fluorphenyl)-3-isoxazolcarboxamid und N-(2-Aminoäthyl)-4-(3-fluorphenyl)-1-methyl-3-pyrazolcarboxamid.

9. Verbindungen der allgemeinen Formel
R-CO-Y
worin R eine der Gruppen

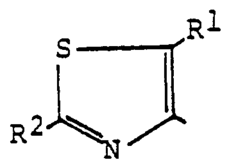

(a)

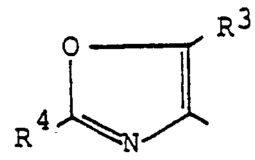

(b)

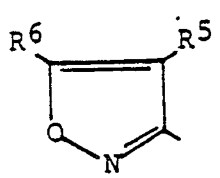

(c)

und

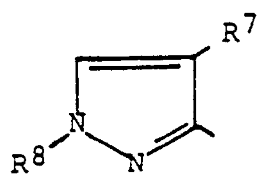

(d)

und Y Hydroxy oder die Gruppe $-NH-CH_2-CH_2-R^{14}$ bedeuten, worin $R^1$ gegebenenfalls durch Halogen, nieder-Alkyl, nieder-Alkoxy, Trifluormethyl, Cyan oder Aryl-niederalkoxy monosubstituiertes Phenyl, Dihalophenyl, Furyl oder gegebenenfalls durch Halogen monosubstituiertes Thienyl, $R^2$ Wasserstoff, Halogen oder Amino, $R^3$, $R^5$ und $R^7$ je gegebenenfalls durch Halogen mono-oder di-substituiertes Phenyl, Thienyl oder gegebenenfalls durch Halogen monosubstituiertes Furyl, $R^4$ und $R^6$ je Wasser stoff oder Amino, $R^8$ Wasserstoff oder nieder-Alkyl und $R^{14}$ eine Abgangsgruppe oder einen in eine Aminogruppe überführbaren Rest bedeuten.

10. Verbindungen gemäss einem der Ansprüche 1-8 sowie pharmazeutisch verwendbare Säureadditionssalze davon zur Anwendung als therapeutische Wirkstoffe.

11. Verbindungen gemäss einem der Ansprüche 1-8 sowie pharmazeutisch verwendbare Säureadditionssalze davon zur Anwendung als Antidepressiva oder Mittel zur Behandlung bzw. Verhütung von kognitiven Erkrankungen.

12. Verfahren zur Herstellung von Verbindungen gemäss einem der Ansprüche 1-8 sowie von pharmazeutisch verwendbaren Säureadditionssalzen davon, dadurch gekennzeichnet, dass man

a) eine Verbindung der allgemeinen Formel
R-CO-OH II
worin R die in Anspruch 1 angegebene Bedeutung hat, in Form der freien Säure oder in Form eines reaktionsfähigen funktionellen Derivates davon mit Aethylendiamin umsetzt, oder

b) eine Verbindung der allgemeinen Formel
$R-CO-NH-CH_2-CH_2-R^9$ III
worin R die in Anspruch 1 angegebene Bedeutung hat und $R^9$ eine Abgangsgruppe bedeutet, mit Ammoniak umsetzt, oder

c) in einer Verbindung der allgemeinen Formel
$R-CO-NH-CH_2-CH_2-R^{10}$ IV
worin R die in Anspruch 1 angegebene Bedeutung hat und $R^{10}$ einen in eine Aminogruppe überführbaren Rest bedeutet,
den Rest $R^{10}$ in die Aminogruppe überführt, und erwünschtenfalls eine erhaltene Verbindung in ein pharmazeutisch verwendbares Säureadditionssalz überführt.

13. Arzneimittel, enthaltend eine Verbindung gemäss einem der Ansprüche 1-8 und ein therapeutisch inertes Excipiens.

14. Antidepressivum oder Mittel zur Behandlung bzw. Verhütung von kognitiven Erkrankungen, enthaltend eine Verbindung gemäss einem der Ansprüche 1-8 und ein therapeutisch inertes Excipiens.

15. Verwendung einer Verbindung gemäss einem der Ansprüche 1-8 zur Herstellung eines Antidepressivums oder eines Mittels zur Behandlung bzw. Verhütung von kognitiven Erkrankungen.